# EUROPÄISCHE PATENTSCHRIFT

(11) **EP 1 399 551 B1**
(45) Veröffentlichungstag und Bekanntmachung des Hinweises auf die Patenterteilung: **02.11.2006**
(21) Anmeldenummer: 02750798.7
(22) Anmeldetag: 24.06.2002
(51) Int. Cl.: C12N 15/10, C12N 15/34, C12N 15/62, C07K 14/01

(54) **VERFAHREN ZUR AUFREINIGUNG VON BAKTERIENZELLEN UND ZELLBESTANDTEILEN**
METHOD FOR SEPARATING BACTERIAL CELLS AND CELL COMPONENTS
PROCEDE DE SEPARATION DE CELLULES BACTERIENNES ET DE COMPOSANTS CELLULAIRES

(30) Priorität: 24.06.2001 DE 10129815
(43) Veröffentlichungstag der Anmeldung: 24.03.2004
(73) Patentinhaber: Profos AG, 93053 Regensburg (DE)
(72) Erfinder: SCHÜTZ,Michael, 93138 Lappersdorf (DE); GRASSL, Renate, 93049 Regensburg (DE); MEYER, Roman, 92287 Schmidmühlen (DE); FRICK, Sibylle, 93197 Zeitlarn (DE); ROBL, Ingrid, 93055 Regensburg (DE); ZANDER, Thomas, 93138 Lappersdorf (DE); MILLER, Stefan, 93053 Regensburg (DE)
(74) Vertreter: Dehmel, Albrecht
(86) Internationale Anmeldenummer: PCT/DE2002/002302
(87) Internationale Veröffentlichungsnummer: WO 2003/000888

(56) Entgegenhaltungen:
- WO-A-00/23792
- WO-A-01/09370
- WO-A-93/17129
- SUN W ET AL: "Use of bioluminescent Salmonella for assessing the efficiency of constructed phage-based biosorbent." JOURNAL OF INDUSTRIAL MICROBIOLOGY & BIOTECHNOLOGY, Bd. 25, Nr. 5, November 2000 (2000-11), Seiten 273-275, XP008016601 ISSN: 1367-5435
- BENNETT A R ET AL: "THE USE OF BACTERIOPHAGE-BASED SYSTEMS FOR THE SEPARATION AND CONCENTRATION OF SALMONELLA" JOURNAL OF APPLIED MICROBIOLOGY, OXFORD, GB, Bd. 83, Nr. 2, 1997, Seiten 259-265, XP001051057 ISSN: 1364-5072
- CAPARON MAIRE H ET AL: "Analysis of novel streptavidin-binding peptides, identified using a phage display library, shows that amino acids external to a perfectly conserved consensus sequence and to the presented peptides contribute to binding." MOLECULAR DIVERSITY, Bd. 1, Nr. 4, 1996, Seiten 241-246, XP000884210 ISSN: 1381-1991
- HAUKANES B-I ET AL: "APPLICATION OF MAGNETIC BEADS IN BIOASSAYS" BIO/TECHNOLOGY, NATURE PUBLISHING CO. NEW YORK, US, Bd. 11, Nr. 1, 1993, Seiten 60-63, XP000609039 ISSN: 0733-222X
- SELIVANOV N A ET AL: "NUCLEOTIDE AND DEDUCED AMINO ACID SEQUENCE OF BACTERIOPHAGE T4 GENE12" NUCLEIC ACIDS RESEARCH, OXFORD UNIVERSITY PRESS, SURREY, GB, Bd. 16, Nr. 5, 1988, Seite 2334 XP001052994 ISSN: 0305-1048
- SAFARIK I.; SAFARIKOVA M.: 'Use of magnetic techniques for the isolation of cells.' J. CHROMATOGRAPHY B Bd. 722, 1999, Seiten 33 - 53

### SEQUENCE LISTING

<110> PROFOS AG
<120> Verfahren zur Aufreinigung von Bakterienzellen und Zellbestandteilen
<130> PRO-004 PCT
<150> DE 101 29 815
<151> 2001-06-24
<160> 8
<170> Patent In version 3.1
<210> 1
   <211> 78
   <212> DNA
   <213> künstlich hergestellte Sequenz
<400> 1
   gaaggaacta gtcatatggc tagctggagc cacccgcagt tcgaaaaagg cgccagtaat 60
   aatacatatc aacacgtt 78
<210> 2
   <211> 54
   <212> DNA
   <213> künstlich hergestellte Sequenz
<400> 2
   acgcgcaaag cttgtcgacg gatcctatca ttcttttacc ttaattatgt agtt 54
<210> 3
   <211> 78
   <212> DNA
   <213> künstlich hergestellte Sequenz
<400> 3
   gaaggaacta gtcatatggc ttgttggagc cacccgcagt tcgaaaaagg cgccagtaat 60
   aatacatatc aacacgtt 78
<210> 4
   <211> 78
   <212> DNA
   <213> künstlich hergestellte Sequenz
<400> 4
   gaaggaacta gtcatatggc tagctggagc cacccgcagt tcgaaaaagg cgcctgtaat 60
   aatacatatc aacacgtt 78
<210> 5
   <211> 19
   <212> PRT
   <213> künstlich hergestellte Sequenz
<400> 5
   Met Ala Ser Trp Ser His Pro Gln Phe Glu Lys Gly Ala Ser Asn Asn
   1 5 10 15
   Thr Tyr Gln
<210> 6
   <211> 19
   <212> PRT
   <213> künstlich hergestellte Sequenz
<400> 6
   Met Ala Cys Trp Ser His Pro Gln Phe Glu Lys Gly Ala Ser Asn Asn
   1 5 10 15
   Thr Tyr Gln
<210> 7
   <211> 19
   <212> PRT
   <213> künstlich hergestellte Sequenz
<400> 7
   Met Ala Ser Trp Ser His Pro Gln Phe Glu Lys Gly Ala Cys Asn Asn
   1 5 10 15
   Thr Tyr Gln
<210> 8
   <211> 539
   <212> PRT
   <213> künstlich hergestellte Sequenz
<400> 8
   Met Ala Ser Trp Ser His Pro Gln Phe Glu Lys Gly Ala Ser Asn Asn
   1               5                   10                  15
   Thr Tyr Gln His Val Ser Asn Glu Ser Arg Tyr Val Lys Phe Asp Pro
               20                  25                  30
   Thr Asp Thr Asn Phe Pro Pro Glu Ile Thr Asp Val Gln Ala Ala Ile
           35                  40                  45
   Ala Ala Ile Ser Pro Ala Gly Val Asn Gly Val Pro Asp Ala Ser Ser
       50                  55                  60
   Thr Thr Lys Gly Ile Leu Phe Leu Ala Thr Glu Gln Glu Val Ile Asp
   65                  70                  75                  80
   Gly Thr Asn Asn Thr Lys Ala Val Thr Pro Ala Thr Leu Ala Thr Arg
                   85                  90                  95
   Leu Ser Tyr Pro Asn Ala Thr Glu Ala Val Tyr Gly Leu Thr Arg Tyr
               100                 105                 110
   Ser Thr Asp Asp Glu Ala Ile Ala Gly Val Asn Asn Glu Ser Ser Ile
           115                 120                 125
   Thr Pro Ala Lys Phe Thr Val Ala Leu Asn Asn Val Phe Glu Thr Arg
   130 135 140
   Val Ser Thr Glu Ser Ser Asn Gly Val Ile Lys Ile Ser Ser Leu Pro
   145 150 155 160
   Gln Ala Leu Ala Gly Ala Asp Asp Thr Thr Ala Met Thr Pro Leu Lys
   165 170 175
   Thr Gln Gln Leu Ala Val Lys Leu Ile Ala Gln Ile Ala Pro Ser Lys
   180 185 190
   Asn Ala Ala Thr Glu Ser Glu Gln Gly Val Ile Gln Leu Ala Thr Val
   195 200 205
   Ala Gln Ala Arg Gln Gly Thr Leu Arg Glu Gly Tyr Ala Ile Ser Pro
   210 215 220
   Tyr Thr Phe Met Asn Ser Thr Ala Thr Glu Glu Tyr Lys Gly Val Ile
   225 230 235 240
   Lys Leu Gly Thr Gln Ser Glu Val Asn Ser Asn Asn Ala Ser Val Ala
   245 250 255
   Val Thr Gly Ala Thr Leu Asn Gly Arg Gly Ser Thr Thr Ser Met Arg
   260 265 270
   Gly Val Val Lys Leu Thr Thr Thr Ala Gly Ser Gln Ser Gly Gly Asp
   275 280 285
   Ala Ser Ser Ala Leu Ala Trp Asn Ala Asp Val Ile His Gln Arg Gly
   290 295 300
   Gly Gln Thr Ile Asn Gly Thr Leu Arg Ile Asn Asn Thr Leu Thr Ile
   305 310 315 320
   Ala Ser Gly Gly Ala Asn Ile Thr Gly Thr Val Asn Met Thr Gly Gly
   325 330 335
   Tyr Ile Gln Gly Lys Arg Val Val Thr Gln Asn Glu Ile Asp Arg Thr
   340 345 350
   Ile Pro Val Gly Ala Ile Met Met Trp Ala Ala Asp Ser Leu Pro Ser
           355                 360                 365
   Asp Ala Trp Arg Phe Cys His Gly Gly Thr Val Ser Ala Ser Asp Cys
       370                 375                 380
   Pro Leu Tyr Ala Ser Arg Ile Gly Thr Arg Tyr Gly Gly Ser Ser Ser
   385                 390                 395                 400
   Asn Pro Gly Leu Pro Asp Met Arg Gly Leu Phe Val Arg Gly Ser Gly
                   405                 410                 415
   Arg Gly Ser His Leu Thr Asn Pro Asn Val Asn Gly Asn Asp Gln Phe
               420                 425                 430
   Gly Lys Pro Arg Leu Gly Val Gly Cys Thr Gly Gly Tyr Val Gly Glu
           435                 440                 445
   Val Gln Lys Gln Gln Met Ser Tyr His Lys His Ala Gly Gly Phe Gly
       450                 455                 460
   Glu Tyr Asp Asp Ser Gly Ala Phe Gly Asn Thr Arg Arg Ser Asn Phe
   465                 470                 475                 480
   Val Gly Thr Arg Lys Gly Leu Asp Trp Asp Asn Arg Ser Tyr Phe Thr
                   485                 490                 495
   Asn Asp Gly Tyr Glu Ile Asp Pro Ala Ser Gln Arg Asn Ser Arg Tyr
               500                 505                 510
   Thr Leu Asn Arg Pro Glu Leu Ile Gly Asn Glu Thr Arg Pro Trp Asn
           515                 520                 525
   Ile Ser Leu Asn Tyr Ile Ile Lys Val Lys Glu
       530                 535

## Beschreibung

Die vorliegende Erfindung betrifft ein Verfahren zur selektiven Aufreinigung von Bakterienzellen und/oder Zellbestandteilen, wobei die Aufreinigung mittels eines festen Trägers durchgeführt wird.

Ausgangspunkt fast jeder Weiterverarbeitung, Analyse oder Isolierung von Zellbestandteilen ist die Anreicherung der Zellen, die "Zellernte". Diese wird üblicherweise mittels Zentrifugation durchgeführt. Dieser Zentrifugationsschritt stellt das Hauptproblem bei der vollständigen Automatisierung von Verfahren, wie der Plasmidaufreinigung dar, da neben dem hohen technischen Aufwand zur Integration einer Zentrifuge in einen entsprechenden Verarbeitungsroboter eine extrem hohe Präzision bei Start- und Stop-Position des Zentrifugationsvorganges benötigt wird. Automatische Verfahren der Weiterverarbeitung, Analyse oder Isolierung von Zellbestandteilen beginnen daher in der Regel mit bereits ausserhalb des Verarbeitungsroboters angereicherten, abzentrifugierten oder sedimentierten Zellen. So ist zum Beispiel für eine schnelle Analyse von vollständigen Genomen, Proteomen, aber auch der raschen Struktur- und Funktionsaufklärung in Hochdurchsatzverfahren eine möglichst vollständige Automatisierung der dabei relevanten Verfahren essentiell. Dabei ist die Automatisierung z.B. in der Genomanalyse bereits weit fortgeschritten: Sowohl die Bakterienanzucht, als auch die Plasmidisolierung können automatisch durchgeführt werden. Eine vollständige Automatisierung des Verfahrens einschließlich der Zellernte ist bisher jedoch nicht durchführbar. Insbesondere eine selektive Zellernte, also die spezifische Anreicherung bestimmter Zellen aus einer Zellmischung ist mit den derzeit üblichen Ernteverfahren nicht möglich.

Üblicherweise wird die Zellernte mit den folgenden Verfahren durchgeführt: Das Standardverfahren der Zellernte ist die unspezifische Abzentrifugation der Bakterienkulturen. Dazu ist gerade bei Verfahren, die für höheren Durchsatz ausgelegt werden sollen, eine Mikrotiterplattenzentrifuge nötig. Jedoch ist die Zentrifugation als solches nicht zur Automatisierung geeignet.

Die Filtration der kultivierten Zellen über entsprechende Filtermembranen ist zwar als solches möglich, jedoch ermöglicht auch diese nur eine unspezifische Anreicherung der Zellen. Darüber hinaus ist das Verfahren bei hochangereicherten Zellsuspensionen und der damit einhergehenden hohen Viskosität der Lösungen sehr störanfällig bezüglich Verstopfungen.

Die fluoreszenzaktivierte Zellsortierung ist ein Verfahren, bei dem ein sehr dünner Flüssigkeitsfaden eingesetzt wird, der über Laser eine Sortierung und Anreicherung einzelner fluoreszenzmarkierter Zellen ermöglicht. Durch den Einsatz entsprechender Fluoreszenzlabel ist zwar eine gewisse Spezifität der Anreicherung möglich, durch den dünnen Flüssigkeitsfaden ist das Verfahren auf geringe Volumina und damit geringen Durchsatz limitiert. Somit könnten nur geringe Zellmengen angereichert werden, was für die Weiterverarbeitung oder Analyse von Zellbestandteilen nicht ausreichend ist. Die hohen Kosten für die apparative Ausstattung verhindert zudem die starke Verbreitung der Technik und bremst die Parallelisierung, die für eine Hochdurchsatzzellemte nötig ist.

Bei der magnetischen Zellseparation werden die Zellen direkt über ionische Wechselwirkungen an Magnetpartikel gebunden und durch Anlegen eines Magnetfeldes lokal aufkonzentriert. Derartige Verfahren zur unspezifischen Konzentrierung von Bakterien werden von Chemagen (EP 1 118 676), Genpoint (WO 01/53525, WO 98/51693), Merck (WO 00/29562), sowie Promega (US 6,284,470) oder Amersham (WO 91/12079) seit kurzem zur vollständigen Automatisierung der Aufarbeitung von Plasmid- oder genomischer DNA inklusive der Zellernte vetrieben. Diese Magnetpartikel haben jedoch den Nachteil, dass sie die Bakterien einerseits unspezifisch und andererseits jedoch nicht jede Bakterienspecies gleich gut binden. Aufgrund der Unspezifität der Bindung sind sogar unterschiedliche Bindeeffizienzen bei verschiedenen Stämmen einer Spezies zu beobachten (Merck, WO 00/29562).

Sun et al. (Journal of Industrial Microbiology & Biotechnology 25 ; 2000, Seiten 273-275) offenbaren mit Streptavidin beschichtete Magnetpartikel, auf deren Oberfläche biotinylierte vollständige Bakteriophagen aufgebracht sind. Dieses System stellt ein Bakteriophagenbasiertes Biosorbens dar, das unmittelbar zur Anreicherung von Bakterien der Spezies *S*. *enteritidis* verwendet werden kann. Ein Nachteil dieses Verfahrens ist, dass vollständige Bakteriophagen verwendet werden, die unter Umständen die aufzureinigenden Bakterienzellen noch lysieren könnten.

Daher liegt der Erfindung die Aufgabe zu Grunde, ein Verfahren bereitzustellen, mit dem Bakterienzellen und Zellbestandteile selektiv und vollautomatisch angereichert werden können, und das in ein automatisiertes Analyse- oder Isolierungsverfahren eingebaut werden kann sowie in der Bereitstellung von festen Trägern zur selektiven Anreicherung von Bakterienzellen oder Zellbestandteilen.

Die Aufgabe wird durch den in den Patentansprüchen definierten Gegenstand gelöst.

Die nachfolgenden Figuren erläutern die Erfindung.

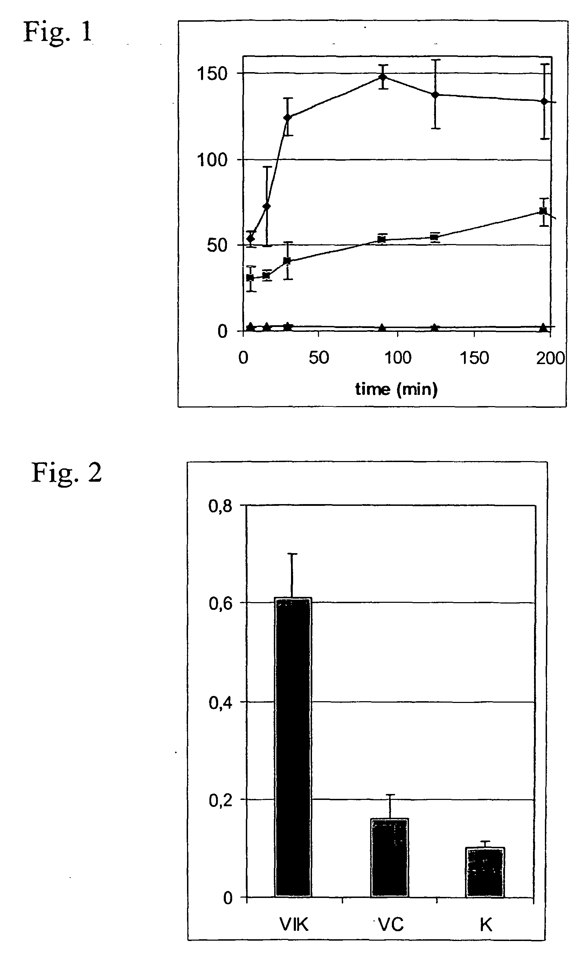

Figur 1 zeigt in einer Graphik die Zeitabhängigkeit der p12-abhängigen Bindung von *E.coli* an magnetische Beads. Die Werte geben die β-Galaktosidase-Aktivität immobilisierter Zellen in relativen Einheiten an. VIK (Rautensymbole) bedeutet: 2-Schritt-Verfahren (Vorinkubation von Zellen und p12, anschliessende Bindung an magnetische Beads). VC (Quadrate) bedeutet: 1-Schritt-Verfahren (Vorcoating von p12 an magnetische Beads, anschliessende Immobilisierung von Zellen). -p12 (Dreiecke) bedeutet: Hintergrund (unspezifische Zellbindung an Beads ohne p12).

Figur 2 zeigt in einer Graphik die Bindung von *E.coli* an magnetische Beads über den Bakteriophagen T4. Die Werte geben die β-Galaktosidase-Aktivität immobilisierter Zellen in relativen Einheiten an. VIK bedeutet: 2-Schritt-Verfahren (Vorinkubation von Zellen und T4, anschliessende Bindung an magnetische Beads). VC bedeutet: 1-Schritt-Verfahren (Vorcoating von T4 an magnetische Beads, anschliessende Immobilisierung von Zellen). K bedeutet: Hintergrund (unspezifische Zellbindung an Beads ohne T4).

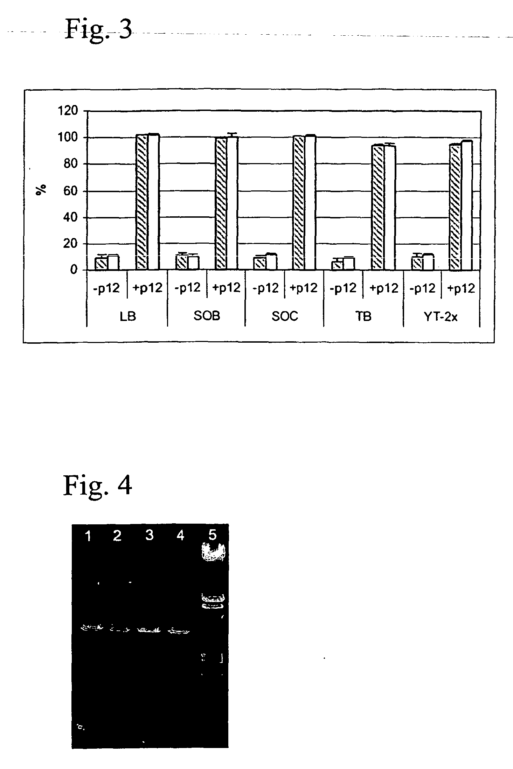

Figur 3 zeigt in einem graphischen Vergleich die Ausbeuten von *E.coli* Zellen aus verschiedenen Medien. -p12 kennzeichnet die Ergebnisse ohne N-Strep-p12. +p12 kennzeichnet die Ergebnisse mit N-Strep-p12. Schraffierte Balken zeigen die Ergebnisse mit dem Stamm *E.coli* LE392, ausgefüllte Balken kennzeichnen die Ergebnisse mit dem Stamm *E.coli* JM83, LB, SOB, SOC, TB und YT 2x bezeichnen die jeweiligen Medien in denen der Versuch durchgeführt wurde. Die Werte sind angegeben als Ausbeuten in % der eingesetzten Zellen ermittelt über die Streuung des Überstandes bei 600 nm nach Pelletierung der gebundenen Zellen mittels eines Magneten.

Figur 4 zeigt graphisch das Ergebnis der Plasmidisolierung nach Ernte von *E.coli* mit p12 nach dem 2-Schritt-Verfahren. Stamm DH10b mit Plasmid pUC19. 1: Zentrifugierte Zellen, DNA-Isolierung über solid phase extraction, 2: Mit dem erfindungsgemäßen 2-Schritt-Verfahren geerntete Zellen, DNA-Isolierung über solid phase extraction, 3: Zentrifugierte Zellen, DNA-Isolierung über magnetische Beads, 4: Mit dem erfindungsgemäßen 2-Schritt-Verfahren geerntete Zellen, DNA-Isolierung magnetische Beads, 5: Standard.

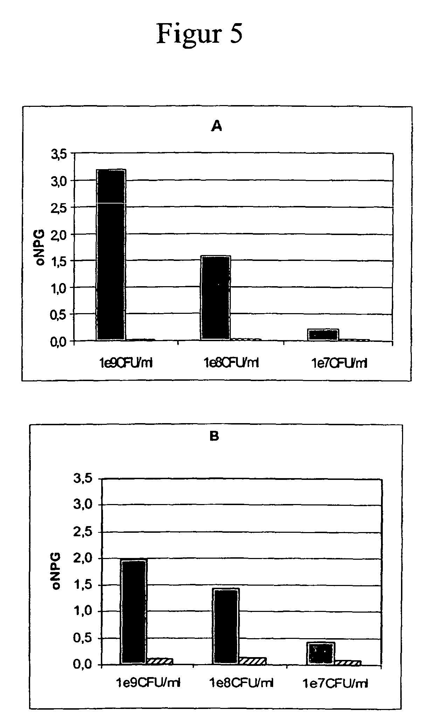

Figur 5 zeigt in einer graphischen Darstellung die Anreicherung von *E.coli* Zellen aus 10ml Kulturvolumen, ausgehend von Zellsuspensionen unterschiedlicher Zelldichte (10⁹-10⁷ CFU/ml). Graph A: die ausgefüllten Balken geben die β-Galaktosidase-Aktivität der über N-Strep-p12 gebundenen Zellen an, die schraffierten Balken zeigen den Hintergrund ohne p12. Graph B: die ausgefüllten Balken geben die β-Galaktosidase-Aktivität der über T4-bio gebundenen Zellen an, die schraffierten Balken zeigen den Hintergrund ohne T4.

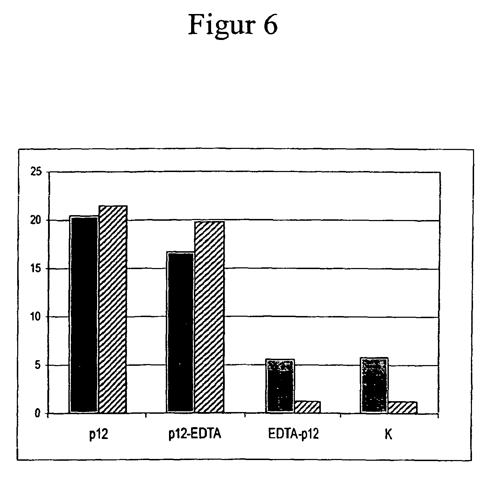

Figur 6 zeigt schematisch die Lebend-Ernte von *E.coli* über biotinyliertes p12 und Streptavidin-Beads. Die ausgefüllten Balken geben die Streuung bei 600 nm nach 2h Wachstum an. Die schraffierten Balken markieren die β-Galaktosidase-Aktivität der Zellen. Die Abkürzungen bedeuten: P12: Bestimmungen der immobilisierten Zellen an den Beads, P12-EDTA: Bestimmungen der von den Beads nach der Bindung abgelösten Zellen (Überstand nach EDTA-Behandlung), EDTA-p12: Immobilisierung der Zellen an die Beads wurde durch die Gegenwart von EDTA verhindert; Bestimmungen der unspezifischen Zellen an den Beads, K. Kontrollversuch ohne p12; Bestimmungen an den Beads.

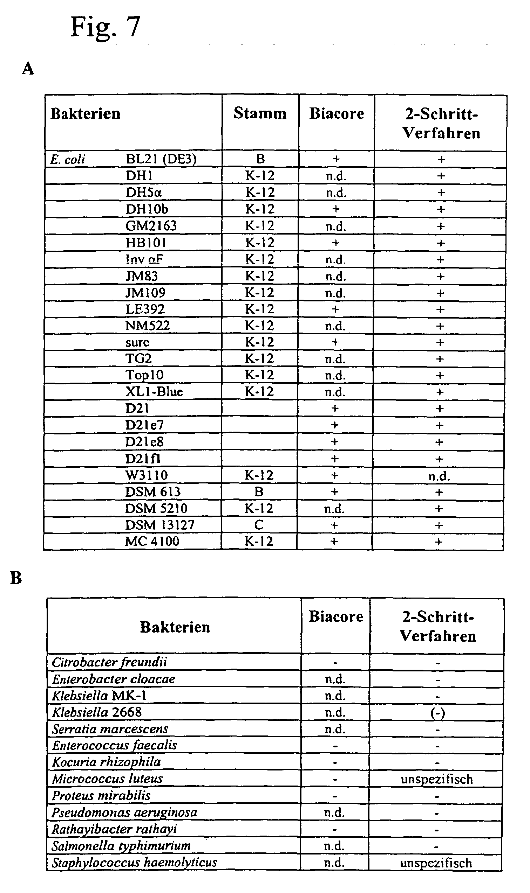

Figur 7 zeigt tabellarisch die selektive Bindung von p12 an Bakterienzellen. Tabelle A listet die p12-abhängige Bindung verschiedener *E.coli* Stämme auf, Tabelle B gibt die Spezifität der p12-abhängigen Bindung an. Die Abkürzung n.d. steht für nicht ermittelt, + bedeutet Bindung von p12 an die angegebenen Bakterien, - bedeutet keine Bindung von p12 an die Zellen.

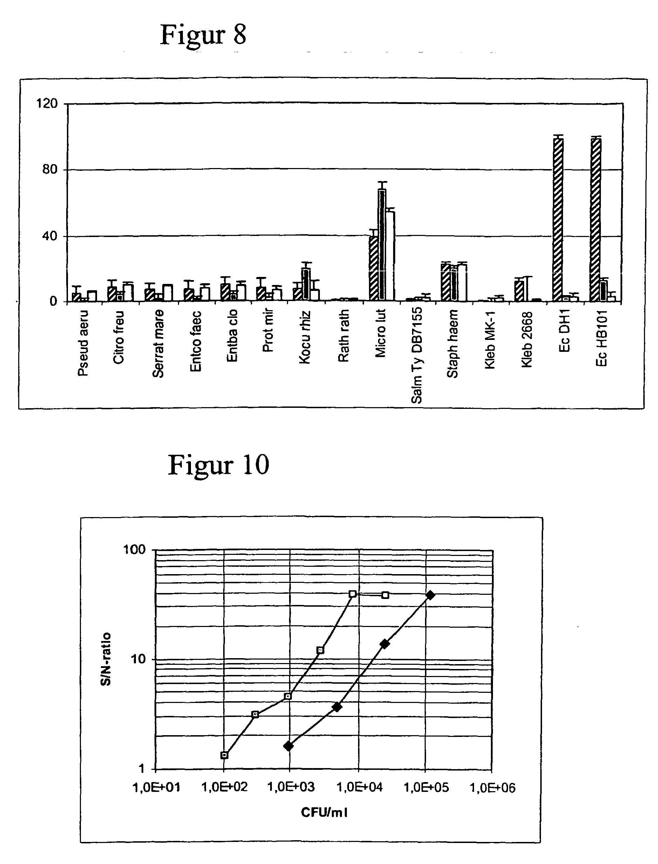

Figur 8 zeigt graphisch dargestellt die Spezifität der p12-abhängigen Bindung. Die Werte geben die Ausbeute von immobilisierten Zellen, ermittelt über die Streuung im Überstand bei 600 nm, an. Die schraffierten Balken geben die Werte für die Zellbindung über N-Strep-p12 an (= spezifische Bindung). Die dunklen ausgefüllten Balken zeigen die Kontrolle ohne p12 (= unspezifische Adsorption). Die hellen ausgefüllten Balken geben die Werte für die Zellbindung mit p12, jedoch in Gegenwart von 10mM EDTA an (= unspezifische Adsorption).

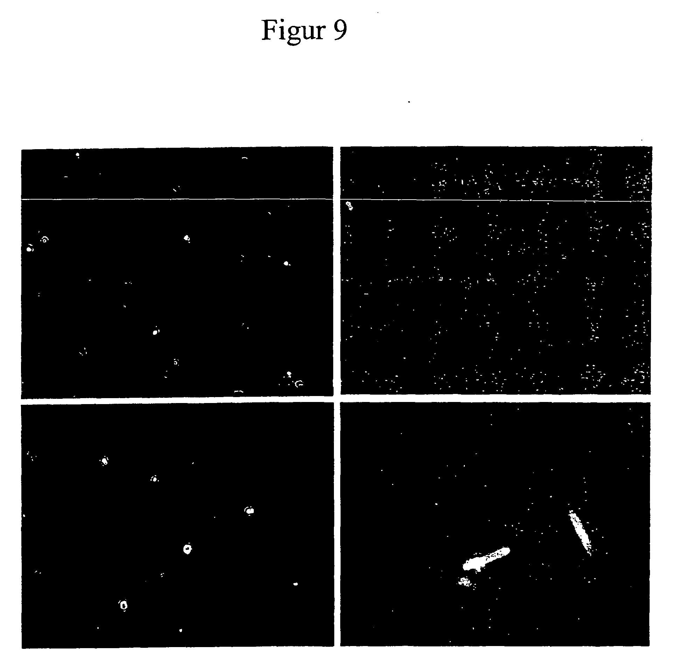

Figur 9 zeigt lichtrnikroskopische und fluoreszenzmikroskopische Aufnahmen der selektiven Bindung von *E.coli* an magnetische Beads über T4-bio in einer Mischkultur aus *E.coli* und *Serratia marrescens.* Die *E.coli* Zellen wurden fluoreszenzmarkiert mit FITC-markiertem T4-p12. Die linken Bilder zeigen lichtmikroskopische Aufnahmen, die rechten Bilder zeigen fluoreszenzmikroskopische Aufnahmen. Die oberen Bilder zeigen Aufnahmen eines Versuchs ohne T4-bio, die unteren Bilder zeigen Aufnahmen eines Versuchs mit T4-bio.

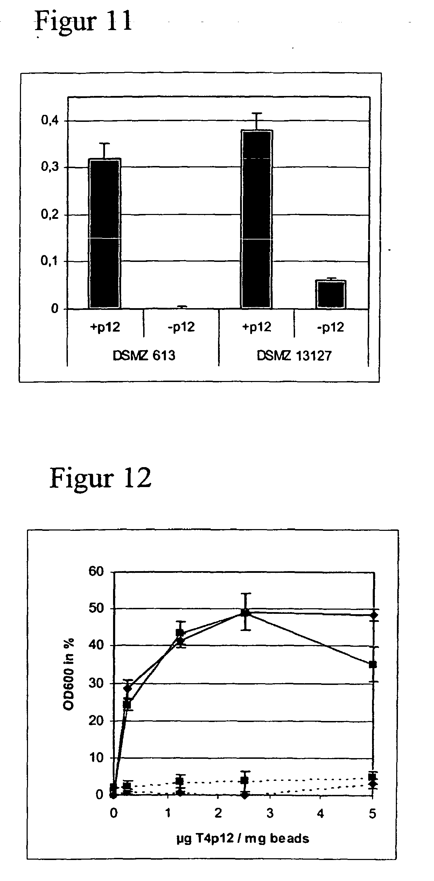

Figur 10 zeigt in einer graphischen Darstellung das Ergebnis der Zellernte nach dem 2-Schritt-Verfahren mit N-Strep-p12 aus Lösungen mit unterschiedlicher Zellzahl. S/N-ratio bezeichnet das Signal-Rausch-Verhältnis (Signal mit N Strep-p12 dividiert durch Signal ohne N-Strep-p12) der β-Galactosidase-Reaktion der gebundenen *E.coli* Zellen, CFU/ml gibt die eingesetzten Zellen (cell forming units) pro ml an. Die Quadrate kennzeichnen die Ergebnisse der β-Galactosidase-Aktivität über Messung mit einem lumineszierenden Substrat. Die Rauten kennzeichnen die Ergebnisse der β-Galactosidase-Aktivität über Messung mit einem fluoreszierenden Substrat.

Figur 11 zeigt in einer graphischen Darstellung das Ergebnis der Ernte von *E.coli* Zellen nach dem 1-Schritt-Verfahren mit T4p12 kovalent gebunden an magnetische EM2-100/49 Beads (Merck Eurolab). +p12 bezeichnet die Ergebnisse mit Beads mit T4p12, -p12 bezeichnet die Ergebnisse mit Beads ohne T4p12, DSMZ 613 bezeichnet die Ergebnisse mit dem Stamm *E.coli* DSMZ 613, DSMZ 13127 bezeichnet die Ergebnisse mit dem Stamm *E.coli* DSMZ 13127.

Figur 12 zeigt in einer graphischen Darstellung das Ergebnis der Ernte von *E.coli* Zellen nach dem 1-Schritt-Verfahren mit T4p12 adsorbiert an magnetische PVA-Beads. Die Rauten kennzeichnen die Ergebnisse mit den Beads PVA-011 (Chemagen). Die Quadrate kennzeichnen die Ergebnisse mit den Beads PVA-012. Durchgehende Linien bezeichnen die Ergebnisse mit Beads an die T4p12 adsorbiert wurde, gestrichelte Linien Beads ohne T4p12. Die OD600 Werte geben die % geernteter Zellen in % der Streuung des Überstandes nach Pelletierung der gebundenen Zellen mittels eines Magneten an.

Der hier verwendete Ausdruck "Phagenproteine" oder "Bakteriophagenproteine" bezeichnet alle Proteine von Bakteriophagen, die an der Erkennung und Bindung der Bakterienzellen oder Zellbestandteilen beteiligt sind. Diese können je nach morphologischer Beschaffenheit der Phagen z.B. direkt auf der Phagenhülle oder auf speziellen Erkennungsstrukturen, den Schwanzfasern lokalisiert sein. Demzufolge bezeichnet der Ausdruck "Bakteriophagenschwanzproteine" die Phagenproteine, die den Bakteriophagenschwanz darstellen oder Teil des Bakteriophagenschwanzes sind.

Der hier verwendete Ausdruck "Spezifität" bedeutet, dass die Bakteriophagen oder Phagenproteine sowohl nur eine einzige Gattung oder Species oder auch Sub-Species von Bakterienzellen oder Zellbestandteile erkennen und binden, als auch, dass manche Bakteriophagen oder Phagenproteine auch bestimmte Bakteriengruppen erkennen und binden.

Der hier verwendete Ausdruck "Aufreinigung" oder "Anreicherung" bedeutet das spezifische Abtrennen von Bakterienzellen oder Zellbestandteilen von der wässrigen Lösung, z.B. dem Kulturmedium, in der sich die Bakterienzellen oder Zellbestandteile befinden. Das Aufreinigen oder Anreichern wird dabei mit Hilfe von festen Trägern z.B. Magnetpartikeln Glaspartikel, Agarosepartikel, Reaktionsgefäßen oder Mikrotiterplatten durchgeführt.

Ein Aspekt der vorliegenden Erfindung besteht in der Bereitstellung eines Verfahrens zur selektiven Aufreinigung von Bakterienzellen oder Zellbestandteilen, umfassend die folgenden Schritte: (Zwei-Schritt-Verfahren)
a) In Kontakt bringen von einer Bakterienzellen oder Zellbestandteile enthaltenden Probe mit Bakteriophagenhüllproteinen und/oder Bakteriophagenschwanzproteinen, vorzugsweise mit einer Inkubation von etwa drei bis fünf Minuten,
b) anschließende Inkubation der Probe, enthaltend die Bakterienzellen oder Zellbestandteile und die Bakteriophagenhüllproteinen und/oder Bakteriophagenschwanzproteinen, mit einem festen Träger, vorzugsweise für etwa drei bis 30 min,
c) Trennen des festen Trägers mit den daran über die Bakteriophagenhüllproteinen und/oder Bakteriophagenschwanzproteinen gebundenen Bakterienzellen oder Zellbestandteilen von der Probe.

Mit dem erfindungsgemäßen Verfahren können Bakterienzellen oder Zellbestandteile selektiv zum Beispiel aus Mischkulturen verschiedener Species oder einer Kultur einer Species angereichert werden. Anzureichernde Zellbestandteile können z.B. Endotoxine, Proteine, Nukleinsäuren oder Saccharide sein. Die Selektivität des Verfahrens wird durch die Auswahl geeigneter Bakteriophagenproteine ermöglicht. Bakteriophagenproteine eignen sich für das erfindungsgemäße Verfahren am besten für eine selektive Anreicherung der Bakterien oder der Zellbestandteile, da Phagen-Bakterien-Systeme in der Natur über lange Zeiträume evolviert sind, so dass die Phagen ihre Wirtsbakterien hochspezifisch und mit hoher Bindungsaffinität erkennen. Für das erfindungsgemäße Verfahren werden vorzugsweise Bakteriophagenproteine verwendet, die für die gewünschten nachzuweisenden Bakterien spezifisch sind. Bakteriophagen wie auch Bakteriophagenproteine haben sich unter widrigen Umweltbedingungen entwickelt, so dass sie stabil gegenüber Einflüssen wie Temperatur-, pH-Schwankungen (Burda et al., Biological Chemistry 2000, 381, 255-258) u.ä. sind und somit in den unterschiedlichsten Aufreinigungspuffern eingesetzt werden können.

Welche Bakteriophagenproteine verwendet werden, hängt davon ab, welche Bakterienarten aufgereinigt werden sollen. Für eine anschliessende Plasmidaufreinigung werden Bakteriophagenproteine bevorzugt, die *E.coli-*Bakterien selektiv binden können, da diese Bakterien die zur Zeit gebräuchlichen für eine Plasmidherstellung sind. Bereits jetzt steht eine große Zahl bekannter Bakteriophagen für einen Großteil der bisher beschriebenen Bakterien zur Verfügung und kann für die selektive Bakterienanreicherung nutzbar gemacht werden. Die nachfolgende Tabelle gibt eine Übersicht von Bakterien und den für sie spezifischen Bakteriophagen, ohne den Anspruch auf Vollständigkeit zu erheben. Die Phagen und die entsprechenden Wirtsbakterien sind u.a. bei folgenden Stammsammlungen erhältlich: ATCC (USA), DSMZ (Deutschland), UKNCC (Großbritannien), NCCB (Niederlande) und MAFF (Japan). Darüber hinaus können Bakteriophagen gegen entsprechende Bakterien bei Bedarf beispielsweise aus Umweltproben einfach nach Standardverfahren (Seeley, N.D. & Primrose, S.B., 1982, J. Appl. Bacteriol. 53, 1-17) isoliert werden.

| **Bakterien:** | **Phagen** |
|---|---|
| Acholeplasma: | 0c1r, 10tur, L2, L51, M1, MVG51, MV-L1, O1, SpV1, V1, V1, V2, V4, V5 |
| Actinomycetes: | 108/016, 119, 29, 37, 43, 51, 59.1, A1-Dat, Aeh2, Bir, M1, MSP8, ø115-A, ø150A, ø31C, P-a-1, PhiC, R1, R2, SK1, SV2, VP5 |
| Actinoplanes/Micromonospora: | Ap3, Ap4, Mm1, Mm3, Mm4, Mm5, phiUW 51 |
| Aeromonas: | 43, 44RR2.8t, 65, Aeh1 |
| Aeromonas hydrophila: | PM1 |
| Agrobacterium: | PIIBNV6, PS8, psi, PT11 |
| Alcaligenes: | 8764, A5/A6, A6 |
| Alteromonas: | PM2 |
| Amycolatopsis: | W11, W2, W4, W7 |
| Bacillus: | 1A, alpha, AP50, BLE, F, G, GA-1, II, IPy-1, mor1, MP13, MP15, ø105, ø29 (phi 29), øNS11, PBP1, PBS1, SP10, SP15, SP3, SP8, SPP1, SPβ, SPy-2, SST, type |
| Bacillus subtilis | 168, W23, SP50, W23, SP01 |
| Bdellovibrio: | MAC-1, MAC-2, MAC-4, MAC-5, MAC-7 |
| Brucella: | Tb |
| Caulobacter: | øCb12r,øCb2, øCb23r, øCb4, øCb5, øCb8r, øCb9, øCP18, øCP2, øCr14, øCr28 |
| Cellulomonas: | O11, O13, O2, O3, O5, O6, O8 |
| Chlamydia: | 1 |
| Chlamydia psittaci: | phi.CPG1 |
| Clostridium: | Ceß, F1, HM2, HM3, HM7 |
| Coryneforme | 7/26, A, A19, AN25S-1, Arp, AS-1, BL3, CONX, MT, N1, øA8010, S-6(L). β |
| Cyanobacteria: | A-4(L), AC-1, LPP-1, S-2L, S-4L, SM-1 |
| *E.coli,* (O15): | P1, T1, Tula, Tulb, Tull |
| *E.coli:* | 1ø3, 1ø7, 1o9, 2D/13, Ae2, alpha10, alpha3, BE/1, BF23, dA, delta1, delta6. do3, do4, do5, Ee9, eta8, f1, fd, G13, G14, G4, G6, HK022, HK97. HR, lambda, M13, M13mp18 , M20, MM, MS2, Mu, O1. ø80, øA, øR, øX174, PA-2, P1, P1D, P2, P22, Qβ, R17, S13. St-1, T1, T2, T3, T4, T5, T6, T7, WA/1 WF/1, WW/1, zeta3, ZG/2, ZJ/2. |
| *E.coli* R1: | C21 |
| *E.coli* O8: | omega 8 |
| *E.coli* (K12): | U3 |
| Enterobacter: | chi, FC3-9, µ2, 01, 11F, 121, 1412, 3, 3T+, 50. 5845, 66F, 7480b, 8893, 9, 9266, a1, alpha 15, b4, B6, B7, Beccles, BZ13, C-1, C16, C2, C-2, DdVI, Esc-7-11, f2, fcan, Fl, Folac; fr, GA, H, H-19J. 12-2, lalpha, ID2, If1, If2, Ike, JP34, JP501, K19, KU1, M, M11, M12, MS2, NL95, ø92, øl, Øii, Omega8, pilHalpha, PR64FS, PRD1, PST, PTB, R, R17, R23, R34, sd, SF, SMB, SMP2, SP, β, ST, tau, tf-1, TH1, TW18, TW28, ViII, VK, W31, X, Y, ZG/1, ZIK/1, ZJ/1, ZL/3, ZS/3 |
| Klebsiella pneumoniae : | AP3, C3: |
| Lactobacillus: | 1b6, 223, fri, hv, hw222a, øFSW, PL-1, y5 |
| Lactococcus lactis: | 1, 643, c2, kh, ml3, PO08, P127, 1358, 1483, 936, 949, BK5-T, c2, KSY1, P001, P008, P107, P335, PO34, PO87 |
| Leuconostoc: | pro2 |
| Listeria: | 4211, |
| Methanothermobacter: | psi M2 (ΨM2) |
| Micrococcus: | N1, N5 |
| Mollicutes: | Br1, C3, L3 |
| Mycobacterium: | 13, lacticola, Leo, ø17, R1-Myb |
| Nocardia/Rhodococcus/Gor dona: | N13, N18, N24, N26, N36, N4, N5 |
| Nocardioides: | X1, X10, X24, X3, X5, X6, D3, D4. |
| Pasteurella: | 22, 32, AU, C-2 |
| Promicromonospora: | P1, P2, P3, P4 |
| Pseudomonas aeruginosa: | Phi CT, phi CTX, PB-1 |
| Pseudomonas: | 12S, 7s, D3, F116, gh-1, gh-1, Kf1, M6, ø1, øKZ, øW-14, Pf1, |
| Pseudonocardia: | W3 |
| Rhizobium: | 2, 16-2-12, 2. 317, 5, 7-7-7, CM1, CT4, m, NM1, NT2, ø2037/1, ø2042, øgal-1-R, WT1 |
| Saccharomonospora: | Mp1, MP2 |
| Saccharothrix: | W1 |
| Salmonella: | epsilon15, Felix 01, 16-19, 7-11, H-19J, Jersey, N4, SasL1, Vil, ZG/3A, San21 |
| Salmonella typhimurium: | A3, A4, P22 |
| Spiroplasma: | 4, C1/TS2 |
| Sporichthya: | Sp1 - |
| Staphylococcus: | 107, 187, 2848A, 3A, 44AHJD, 6, 77, B11-M15, Twort |
| Streptococcus: | 182, 2BV, A25, A25-24, A25-omega8, A25-PE1, A25-VD13, CP-1, Cvir, H39 |
| Streptomyces: | P23, P26, phi A.streptomycini III, phi8238, phiC31, S1, S2, S3, S4, S6, S7, SH10 |
| Terrabacter: | Tb1, Tb2 |
| Tsukamurella: | Ts1 |
| Vibrio: | 06N-22P, 06N-58P, 06N-58P, 4996, alpha3alpha, I, II, III, IV, kappa, nt-1, OXN-100P, OXN-52P, v6, Vf12, Vf33, VP1, VP11, VP3, VP5, X29 |
| Xanthomonas: | Cf, Cf1t, RR66, |
| Yersinia: | 8/C239, phiYeO3-12, YerA41 |

Werden einzelne Phagenproteine verwendet, so bieten diese den Vorteil, dass hier die Eigenschaften eines einzelnen Proteins, statt eines Komplexes aus Proteinen und Nukleinsäuren genutzt werden können. Phagenproteine sind sehr stabil (Burda et al., Biological Chemistry 2000, 381, 255-258); die Stabilität eines Einzelproteins ist wesentlich einfacher zu steuern als die eines Proteinkomplexes. Wichtig ist auch die im Gegensatz zu kompletten Phagen leichtere Modifizierbarkeit (gentechnisch, aber auch chemisch), z.B. der Einbau von "Tags". Darüberhinaus bietet der Einsatz von Phagenproteinen Vorteile bei bestimmten Anschlußverfahren, wie z.B. der Isolierung von Nukleinssäuren (Plasmid-DNA, RNA, genomischer DNA), da im Gegensatz zum Einsatz kompletter Phagen keine Nukleinsäureverunreinigung möglich ist.

Bevorzugt sind Phagenschwanzproteine aus der Familie der Myoviridae, der Podoviridae und Siphoviridae, insbesondere kurze Phagenschwanzproteine, insbesondere die kurzen Phagenschwanzproteine der "geradzahligen T-Phagen" z.B. von T4, T2 oder K3, insbesondere das Bakteriophagenschwanzprotein p12 aus T4, p12 aus T2 (GenBank Accession Nummer X56555), p12 aus K3 (vgl. Burda et al., 2000, Biol. Chem., Vol. 381, pp. 255 - 258) oder die Bakteriophagenschwanzproteine der Phagen Felix 01, P1 oder PB1. Dabei binden zum Beispiel die kurzen Bakteriophagenschwanzproteine der Phagen T4 (p12) und von P1 an Coliforme, das kurze Phagenschwanzprotein von Felix 01 an Salmonellen und das kurze Phagenschwanzprotein von PB1 an Pseudomonaden.

Phagenschwanzproteine wie p12 oder das P22 tailspike Protein weisen eine hohe Stabilität gegenüber Proteasen, Detergentien, chaotropen Agentien z.B. Harnstoff oder Guanidiniumhydrochlorid oder erhöhter Temperatur auf (Goldenberg, D. und King, J.; Temperature-sensitive mutants blocked in the folding or subunit assembly of the bacteriophage P22 tail spike protein. II. Active mutant proteins matured at 30 °C, 1981, J. Mol. Biol. 145, 633-651. Miller, S., Schuler, B. und Seckler, R.; Phage P22 tailspike protein: Removal of headbinding domain unmasks effects of folding mutations on native-state thermal stability, 1998, Prot. Sci. 7, 2223-2232.;Miller, S., Schuler, B. und Seckler, R.; A reversibly unfolding fragment of P22 tailspike protein with native structure: The isolated β-helix domain, 1998, Biochemistry 37, 9160-9168.; Burda et al., 2000, Biol. Chem., Vol. 381, pp. 255 - 258). Die Entfernung der Phagenkopf- bzw. -Basisplattenbindungsregion dieser Proteine kann eine eventuell bestehende Aggregationssensitivität reduzieren. Interessanterweise sind Einzeldomänen bzw. Untereinheiten dieser Proteine deutlich weniger stabil als die intakten oder nur geringfügig verkürzten Trimere (Miller et al., Prot. Sci. 1998; 7: 2223-2232. Phage P22 tailspike protein: Removal of headbinding domain unmasks effects of folding mutations on native-state thermal stability.; Miller-S, et al., Biochemistry 1998; 37: 9160-9168. A reversibly unfolding fragment of P22 tailspike protein with native structure: The isolated β-helix domain), oder aufgrund ihre dreidimensionalen Struktur vermutlich kaum stabil und funktionell exprimierbar: Kristallographische Arbeiten hatten darüber hinaus gezeigt, das Phagenschwanzproteine und Virusrezeptorproteine oft als stark verdrillte Trimere vorliegen wobei der C-Terminus zurückfalten kann, ein Mechanismus der möglicherweise zusätzlichen Schutz vor Proteasen gewährleistet (Mitraki A, Miller S, van Raaij MJ. Review: conformation and folding of novel Beta-structural elements in viral fiber proteins: the triple Beta-spiral and triple Beta-helix. J Struct Biol. 2002 137(1-2):236-247). Darüberhinaus liegen diese Proteinen im nativen Zustand als Homotrimere vor. Mit drei Bindungsstellen tragen die Trimere über eine Erhöhung der Avidität zu einer festeren Bindung von Bakterien bei.

Wie die Bakteriophagenschwanzproteine an die einzelnen Bakterien binden, soll hier beispielhaft an den "geradzahligen T-Phagen" (z.B. T4, T2, K3) verdeutlicht werden. In dieser Gruppe gibt es auf Wirtsseite zwei Komponenten, die von den Phagen erkannt werden: Erstens ein Oberflächenprotein, das spezifisch für individuelle Phagen ist, zweitens das Lipopolysaccharid (LPS), das alle gram-negativen Bakterien in abgewandelter Form auf ihrer Aussenseite umgebungsexponiert besitzen. Die langen Schwanzfasern der "geradzahligen T-Phagen" spielen eine Rolle bei der spezifischen Erkennung der Wirtsbakterien, während das LPS als Rezeptor für die kurzen Schwanzfasern dient. Beim *E.coli* Phagen T4 ist bekannt, dass die durch die langen Schwanzfasern vermittelte spezifische Interaktion mit dem Wirtsbakterium irreversibel wird, sobald auch die kurzen Schwanzfasern an die Bakterienoberfläche gebunden haben. Die kurze Schwanzfaser ist nicht verantwortlich für die genaue Spezifität innerhalb der Wirtsbakteriengruppe und kann deshalb zwischen den verschiedenen "geradzahligen T-Phagen" ausgetauscht werden.

Bakteriophagenschwanzproteine können leicht rekombinant in großen Mengen produziert und unter Verwendung geeigneter Tags oder über einfache chromatographische Standardtrennverfahren aufgereinigt werden. Phagen wie Wirtsstämme sind größtenteils über Stammsammlungen kommerziell erhältlich, bzw. mit einfachen Mitteln isolierbar. Für das erfindungsgemäße Verfahren können jedoch nicht nur die natürlicherweise vorkommenden Bakteriophagenschwanzproteine verwendet werden, sondern auch deren Varianten. Varianten bedeutet im Sinne der vorliegenden Erfindung, dass die Bakteriophagenschwanzproteine eine veränderte Aminosäuresequenz aufweisen. Diese können durch Screening der natürlich auftretenden Varianten, oder durch Zufalls-Mutagenese oder gezielte Mutagenese, aber auch durch chemische Modifikation erhalten werden. Die für das erfindungsgemäße Verfahren verwendeten Bakteriophagenschwanzproteine können durch eine gezielte oder zufällige Mutagenese in ihrer Wirtsspezifität bzw. ihren Bindungseigenschaften an die Trägerstrukturen angepasst werden. Durch die Mutagenese werden Mutationen eingeführt, die Aminosäureadditionen, -deletionen, -substitutionen oder chemische Modifikationen sein können. Diese Mutationen bewirken eine Veränderung der Aminosäuresequenz in der Bindungsregion der Phagen oder Phagenproteine, mit dem Ziel, Spezifität und Bindungsaffinität an Testbedürfnisse anzupassen, z.B. die Bindung der Bakterien an die isolierten Phagenproteine zu erhöhen oder irreversibel zu machen, um die Waschmöglichkeiten zu verbessern. Darüber hinaus kann eine gentechnische oder biochemische Modifikation der Phagenproteine durchgeführt werden, mit dem Ziel, die gegebenenfalls vorhandene enzymatische Aktivität auszuschalten, um dadurch die Bindung zu verbessern oder irreversibel zu machen.

Zur Bindung der aufzureinigenden Bakterien und/oder Zellbestandteilen an die Bakteriophagenhüllproteine und/oder Bakteriophagenschwanzproteine im Zwei-Schritt-Verfahren wird die Probe, z. B. eine Übernachtkultur mit den Bakteriophagenhüllproteine und/oder Bakteriophagenschwanzproteinen in Kontakt gebracht und vorzugsweise inkubiert. Die Inkubation erfolgt bei einer Temperatur im Bereich von 4° bis 90°C, vorzugsweise bei einer Temperatur im Bereich von 4° bis 45°C, besonders bevorzugt bei einer Temperatur im Bereich von 15° bis 37°C, insbesonders bevorzugt bei einer Temperatur im Bereich von 20° bis 37°C, insbesondere bei RT, für bis zu 6 Stunden, vorzugsweise bis zu 4 Stunden, besonders bevorzugt 2 Stunden, insbesondere 1 Stunde, insbesondere bevorzugt 1 bis 20 Minuten, ganz besonders bevorzugt 3 bis 5 Minuten. Beispielsweise kann die Inkubation 2 bis 120 min bei 4° bis 37°C, bevorzugt für 20 bis 30 min bei 25°C oder 37°C, besonders bevorzugt für 3 bis 5 min bei 37°C erfolgen.

Anschließend wird die Probe mit festen Trägern in Kontakt gebracht und inkubiert. Feste Träger können z.B. Magnetpartikel (paramagnetisch oder ferromagnetisch), Glaspartikel, Agarosepartikel, Luminexpartikel, Reaktionsgefäße oder Mikrotiterplatten sein.

Werden Magnetpartikel verwendet, werden diese anschließend der Probe zugegeben. Die Magnetpartikel binden den Bakteriophagenprotein-Bakterien/Zellbestandteil-Komplex, der dann einfach mittels einer Magnetvorrichtung von der Probe abgetrennt und aufgereinigt werden kann. Die Magnetvorrichtung kann dabei an der Außenseite des Gefäßes positioniert sein und entweder zur Anreicherung eingeschaltet werden, so dass die Magnetpartikel sich an der Gefäßwand sammeln, oder an der Gefäß Außenseite entlanggleiten, so dass die Magnetpartikel sich z. B. am Boden des Gefäßes sammeln. Bevorzugt ist die Anreicherung mit einem Permanentmagneten. Die Magnetvorrichtung kann auch in das Gefäß und in die Probe eintauchen, so dass die Magnetpartikel sich an der Magnetvorrichtung niederschlagen (die Magnetvorrichtung kann dabei durch eine Pipettenspitze oder ein vergleichbares Disposable bedeckt sein). Im Gegensatz zu Zentrifugations- oder Filtrationstechniken unterliegen die Bakterien nur minimalen Scherkräften und können somit mit hoher Ausbeute bei Bedarf auch aktiv/lebend angereichert werden. Die einfache Handhabung erleichert einfache schnelle Puffer-/Lösungswechsel und kann sowohl leicht im großen Maßstab durchgeführt werden, wie auch gut automatisiert werden.

Die Magnetpartikel haben einen Durchmesser, der es erlaubt eine ausreichende Menge an Zellen oder Zellbestandteilen je Partikel zu binden. Vorzugsweise haben die Magnetpartikel einen Durchmesser im Bereich von etwa 0,5 bis etwa 4 µm, insbesondere im Bereich von etwa 0,5 bis etwa 2 µm, besonders bedorzugt im Bereich von etwa 0,8 bis etwa 1,8 µm, ganz besonders bevorzugt von etwa 1 µm.

Die Bindung des Bakteriophagenprotein-Bakterien/Zellbestandteil-Komplexes an die festen Träger z.B. Magnetpartikel erfolgt vorzugsweise über geeignete Kopplungsgruppen insbesondere Polypeptide und/oder niedermolekulare Substanzen. Diese Polypeptide können aber auch für die Bakteriophagenproteine spezifische Antikörper, Lektine, Rezeptoren oder Anticaline sein. Ferner können die Bakteriophagenproteine mit niedermolekularen Substanzen z.B. Biotin verknüpft sein, um über diese niedermolekularen Substanzen an Polypeptide z. B. Streptavidin zu binden, die ihrerseits auf dem Träger immobilisiert wurden. Statt Biotin kann ferner der sogenannte Strep-Tag (Skerra, A. & Schmidt, T. G. M. Biomolecular Engineering 16 (1999), 79-86) verwendet werden, der eine kurze Aminosäuresequenz ist und an Streptavidin bindet. Ferner kann der His-Tag verwendet werden, der über zweiwertige Ionen (Zink oder Nickel) oder einen für ihn spezifischen Antikörper (Qiagen GmbH, Hilden) an ein Trägermaterial binden kann. Der Strep-Tag sowie der His-Tag wird vorzugsweise über DNA-Rekombinationstechnologie an die rekombinant hergestellten Bakteriophagenproteine gebunden. Diese Kopplung kann gerichtet, z.B. am N- oder C-Terminus erfolgen. Da für eine effektive Anreicherung insbesondere im Zwei-Schritt-Verfahren eine hohe Bindungskonstante essentiell ist, wird die Kopplungskombination Biotin/Streptavidin mit einem kD von ~10⁻¹⁵M (Gonzales et al. J. Biol. Chem., 1997, 272 (17), pp. 11288-11294) insbesondere bevorzugt. Es hat sich gezeigt, dass diese nicht-kovalente Bindungskombination besser als die zur Verfügung stehenden Antikörper, Anticaline, Rezeptoren und Lektine ist.

Zur Bindung des Komplexes werden die Magnetpartikel mit dem Bakteriophagenprotein-Bakterien/Zellbestandteil-Komplex in Kontakt gebracht und vorzugsweise inkubiert. Die Inkubation erfolgt bei einer Temperatur im Bereich von 4° bis 90°C, vorzugsweise bei einer Temperatur im Bereich von 4° bis 45°C, besonders bevorzugt bei einer Temperatur im Bereich von 15° bis 37°C, insbesondere bevorzugt bei einer Temperatur im Bereich von 20° bis 37°C, insbesondere bei RT, für bis zu 6 Stunden, vorzugsweise bis zu 4 Stunden, besonders bevorzugt 2 Stunden, insbesondere 1 Stunde, insbesondere bevorzugt 1 bis 20 Minuten, ganz besonders bevorzugt 3 bis 5 Minuten. Beispielsweise kann die Inkubation 2 bis 120 min bei 4° bis 37°C, bevorzugt für 20 bis 30 min bei 25°C oder 37°C, besonders bevorzugt für 3 bis 5 min bei 37°C erfolgen.

Das erfindungsgemäße Verfahren wird mit anderen festen Trägern, die zur Probe zugegeben werden können, entsprechend durchgeführt. Die einzelnen. Inkubationsbedingungen und Abtrennschritte müssen für die verschiedenen festen Träger entsprechend angepasst werden. Dies kann einfach durch eine Versuchsreihe durchgeführt werden und bedarf für den Durchschnittsfachmann hier keiner weiteren Erläuterung.

Alternativ kann das Zwei-Schritt-Verfalren auch in entsprechend beschichteten festen Trägern durchgeführt werden, die nicht zur Probe gegeben werden, sondern wobei die Probe auf oder in den festen Träger, z.B. eine Mikrotiterplatte oder ein Reaktionsgefäß gegeben wird. Dabei wird die Probe nach Schritt a) z.B. in die entsprechenden Vertiefungen der Mikrotiterplätte gegeben und dort inkubiert, vorzugsweise für etwa 20 bis 30 Minuten bei den ansonsten gleichen Bedingungen wie vorstehend beschrieben. Die Vertiefungen einer Mikrotiterplatte oder die inneren Wände eines Reaktionsgefäßes können die gleichen Beschichtungen aufweisen wie vorstehend für die Magnetpartikel beschrieben.

Die erfindungsgemäßen Verfahren (Zwei-Schritt-Verfahren) können zum Beispiel als Zentrifugationsersatz verwendet werden, und somit erstmalig die automatische Aufreinigung von Bakterienzellen ermöglichen. Damit ist erstmalig eine vollständige Automatisierung zum Beispiel der Genomanalyse möglich, d. h. vom Animpfen der Bakterienkulturen bis zur Ermittlung der Sequenz. Ferner können die erfindungsgemäßen Verfahren zum Beispiel zur Isolierung von Zellbestandteilen, insbesondere von Lipopolysacchariden, Endotoxinen oder Exopolysacchariden verwendet werden.

Die nachstehenden Ausführungen über die Kopplung oder Immobilisierung von Bakteriophagenproteinen an die Magnetpartikel gelten entsprechend auch für die Kopplung oder Immobilisierung von Bakteriophagenproteinen und Polypeptiden an die festen Träger (Zwei-Schritt-Verfahren). Die Beschichtung der festen Träger mit den vorstehend beschriebenen Polypeptiden oder den Bakteriophagenproteinen kann auf verschiedene Weise erfolgen.

Die Bakteriophagenproteine können über kovalente Kopplung an die festen Träger fixiert werden. Die damit mögliche sehr feste Bindung an den Träger ermöglicht den Einsatz harscher Waschbedingungen für das für die Weiterverarbeitung der angereicherten Zellen evtl. erforderliche Waschen der Zellen. Die Kopplung der Bakteriophagenproteine über Adsorption ist ein sehr einfaches und kostengünstiges Verfahren. Durch Kopplung der Bakteriophagenproteine über Biotin/Streptavidin oder vergleichbare Ligand/Rezeptor-Systeme ist das Zwei-Schritt-Verfahren möglich. Das in diesem Ansatz verwendete Streptavidin kann sowohl über Adsorption als auch über chemische Kopplung fixiert sein. Wichtig bei der Beschichtung ist eine funktionelle Immobilisierung, d.h. die Bakteriophagenproteine verfügen trotz Bindung an die festen Träger über für Bakterien zugängliche Strukturen.

Bei der kovalenten Kopplung können die Bakteriophagenproteine z.B. über primäre Aminogruppen oder Carboxylgruppen an bereits vom Hersteller aktivierte Trägermaterialien, z.B. Magnetpartikel von Merck, Estapor, etc über Standardbedingungen, z.B. -NH₂ über Cyanurylchlorid (Russian Chemical Rev., 1964, 33: 92-103), oder -COO⁻ über EDC (1-Ethyl-3'[3'Dimethylaminopropyl]carbodiimid) (Anal. Biochem. 1990, **185**: 131-135) gekoppelt werden. Darüberhinaus können die festen Träger mit geeigneten Verfahren direkt aktiviert werden. Weiterhin kann die Kopplung gerichtet über Maleimid- oder Iodoacetyl-Spacer an z.B. ein N-Terminal eingeführtes Cystein erfolgen.

Die Immobilisierung der Bakteriophagenproteine an das Trägermaterial mittels Adsorption kann durch Inkubation einer Bakteriophagenproteine-Lösung in wässrigem Puffer, z.B. 100mM Tris pH 7.3 oder 100mM Natriumphosphat pH 7.5, PBS (10 mM Natriumphosphat pH 7.4, 150 mM Natriumchlorid), über mehrere Stunden oder über Nacht bei 4°C bis 45°C, vorzugsweise bei 15°C bis 37°C, besonders bevorzugt bei 20°C bis 37°C, insbesondere bevorzugt bei 37°C oder RT, insbesondere bevorzugt bei 30-65°C für 2-4 h durchgeführt werden. Nach der Adsorption wird die Beschichtungslösung abgenommen und die Trägerstruktur in wässriger, ggfs. gepufferter Lösung gelagert.

Ein weiterer Aspekt der Erfindung ist ein fester Träger, insbesondere ein Magnetpartikel oder eine Mikrotiterplatte, entweder beschichtet mit Bakteriophagenproteinen oder gegen Bakteriophagenproteine gerichteten Polypeptiden. Diese Polypeptide können für die Bakteriophagenproteine spezifische Antikörper, Lektine, Rezeptoren oder Anticaline sein. Ferner können die festen Träger mit Streptavidin beschichtet sein.

Ein weiterer Aspekt der Erfindung sind Bakteriophagenproteine, mit einem Strep-Tag, der ein oberflächenexponiertes Cystein zur spezifischen, gerichteten Biotinylierung aufweist, z.B. die Tags gemäß SEQ ID NO:5, 6 und 7. Ein Beispiel für ein p12 mit Tag ist die in SEQ ID NO:8 aufgeführte Aminosäuresequenz. Bevorzugt ist ein p12 mit einem Tag, insbesondere mit einem Tag mit einem oberflächenexponierten Cystein, insbesondere ein p12 mit dem Tag gemäß SEQ ID NO: 6 und 7. Diese gerichtete Biotinylierung kann zusätzlich durch einen geeigneten Spacer oder Linker vermittelt werden. Ein weiterer Aspekt der Erfindung ist die Nukleinsäuresequenz, die ein Bakteriophagenprotein zusammen mit dem Strep-Tag codiert. Ferner betrifft die vorliegende Erfindung die Aminosäuren mit einer Sequenz gemäß SEQ ID NO:5, 6 und 7. Ferner betrifft die vorliegende Erfindung die Nukleinsäuren, codierend die Aminosäuresequenz gemäß SEQ ID NO:5, 6 und 7.

Ein weiterer Aspekt der Erfindung betrifft einen Kit zur Anreicherung von Bakterienzellen und/oder Zellbestandteilen, umfassend die erfindungsgemäßen festen Träger, z.B. die Magnetpartikel, Glaspartikel, Agarosepartikel, Reaktionsgefäße oder Mikrotiterplatten sowie die für die Anreicherung der Bakterien und/oder Zellbestandteile notwendigen Lösungen mit den Testreagentien.

Der Kit für eine Anreicherung mit Magnetpartikeln enthält vorzugsweise eine stabilisierte Lösung aus einer p12-Variante mit N-terminal eingeführtem Cysteinrest zur gerichteten Biotinylierung, bzw. NS-T4p12 (oder T4p12-bio) 1mg/ml in 100 mM TrisHCl pH8, 150 mM NaCl, 1 mM EDTA, 0.05 % Tween20 supplementiert mit Proteaseinhibitormix (Sigma) als Lösung (bevorzugte Lagerung bei 4°C) oder als Lyophilisat. Ferner eine Partikellösung bestehend aus Streptavidin- oder Streptaktin-beschichteten Magnetpartikeln in stabilisierender Lösung (PBST mit Natriumazid 0.005%).

Der Kit für eine Anreicherung mit Mikrotiterplatten enthält vorzugsweise eine stabilisierte Lösung aus einer p12-Variante mit N-terminal eingeführtem Cysteinrest zur gerichteten Biotinyleirung, bzw. NS-T4p12 (oder T4p12-bio) 1mg/ml in 100 mM TrisHCl pH8, 150 mM NaCl, 1 mM EDTA, 0.05 % Tween20 supplementiert mit Proteaseinhibitormix (Sigma) als Lösung (bevorzugte Lagerung bei 4°C) oder als Lyophilisat. Ferner eine Streptavidin- oder Streptaktin-beschichtete Mikrotiterplatte.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.
1. Die Reinigung von Wildtyp T4 p12 erfolgte nach der von Burda, M.R. & Miller, S. Eur J Biochem. 1999 265 (2), 771-778 beschriebenen Vorschrift.
2. Konstruktion von p12 mit N-terminalem Strep-Tag (N-Strep-p12): Mittels PCR wurde an das 5'-Ende des T4p12-Gens die Nukleotidsequenz für den Strep-Tag (US patent 5,506,121) eingeführt. Hierfür wurde für das 5'-Ende des p12-Gens ein Primer konstruiert (5'-GAA GGA ACT AGT CAT ATG *GCT AGC TGG AGC CAC CCG CAG TTC GAA AAA GGC GCC* AGT AAT AAT ACA TAT CAA CAC GTT-3' (SEQ ID NO:1), der die Nukleotidsequenz des Strep-Tags an seinem 5'-Ende beinhaltet (kursiv in der Sequenz) und eine Restriktionsschnittstelle (*Nde*I*,* unterstrichen in der Sequenz) derart besitzt, dass das Gen im richtigen Leseraster in das Expressionsplasmid eingesetzt werden kann. Für das 3'-Ende des p12-Gens wurde ein Primer konstruiert, der hinter dem p12-Gen eine *Bam*H I Restriktionsschnittstelle (kursiv in der Sequenz) einführt (5'-ACG CGC AAA GCT TGT CGA CGG ATC CTA TCA TTC TTT TAC CTT AAT TAT GTA GTT-3'), (SEQ ID NO:2). Die PCR wurde mit 40 Cyclen (1 min 95°C, 1 min 45°C und 1 min 72°C) durchgeführt. Der PCR-Ansatz wurde mit den Restriktionsendonukleasen *Nde*I und *Bam*HI geschnitten und das gewünschte Fragment nach Größenfraktionierung über ein Agarosegel und Elution aus dem Gel in die *Nde*I und *Bam*HI site des Expressionsplasmids pET21a (Novagen, Merck Eurolab, Darmstadt) eingesetzt. Die Sequenz des N-Strep-p12-Gens wurde über DNA-Sequenzierung auf seine Richtigkeit hin überprüft. Die weiteren Schritte zum Plasmid pNS-T4p12pS7 wurden wie von Burda, M.R. & Miller, S. (Eur J Biochem. 1999 265 (2), 771-778) für T4p12p57 beschrieben durchgeführt. Das Plasmid pNS-T4p12p57 wurde dann in den Expressionsstamm BL21 (DE3) (Novagen, Merck Eurolab, Darmstadt) transformiert.
3. Einfügen eines N-terminalen Cysteinrests in N-Strep-p12 (N-Strep-S3C-p12 und N-Strep-S14C-p12): Die Einfügung eines N-terminalen Cysteinrestes (fett markiert) wurde wie unter 2. beschrieben durchgeführt, wobei dafür zwei neue Primer für das 5'-Ende konstruiert wurden. Für das N-Strep-S3C-p12 wurde der Primer 5'-GAA GGA ACT AGT CAT ATG *GCT **TGT** TGG AGC CAC CCG CAG TTC GAA AAA GGC GCC* AGT AAT AAT ACA TAT CAA CAC GTT-3' (SEQ ID NO:3), für das N-Strep-S14C-p12 wurde der Primer 5'-GAA GGA ACT AGT CAT ATG *GCT AGC TGG AGC CAC CCG CAG TTC GAA AAA GGC GCC* **TGT** AAT AAT ACA TAT CAA CAC GTT-3' (SEQ ID NO:4) verwendet.
4. Reinigung von N-Strep-p12 Protein: Der *E.coli* Stamm BL21 (DE3) mit dem Plasmid pNS-T4p12p57 wurde in 2 l Schüttelkulturen (LB-Medium mit Ampicillin 100 µg/ml) bis zu einer OD600 von 0.5-0.7 bei 37°C gezogen und die Expression des N-Strep-p12-Proteins wurde durch Zugabe von 1mM IPTG (Isopropyl-β-thio-galactopyranoside) induziert. Nach Inkubation bei 37°C für 4h wurden die Zellen abgeerntet. Geerntete Zellen aus 10 l Kultur wurden in 50 ml Natriumphosphat, 20 mM pH 7.2, 2 mM MgSO4, 0.1 M NaCl aufgenommen, durch dreimalige French-Press-Behandlung (20.000 psi) aufgebrochen und anschließend 30 min bei 15.000 rpm (SS34) abzentrifugiert. Nach zweimaligem Waschen im gleichen Puffer wurde das N-Strep-p12 Protein aus dem Pellet durch Rühren für 30 min in 40 mM TrisHCl pH 8.0, 10 mM EDTA extrahiert, der Ansatz für 30 min bei 15.000 rpm (SS34) zentrifugiert und das abgelöste NS-p12 im Überstand bei 4°C gelagert. Die Extraktion wurde zweimal wiederholt. und die vereinigten Überstände wurden auf eine Streptactin-Affinitätssäule (15 ml), äquilibriert mit Puffer "W" (100 mM TrisHCl pH 8, 1 mM EDTA, 150 mM NaCl), aufgetragen (IBA GmbH, Göttingen). Nach Waschen mit 5 Säulenvolumina Puffer "W" wurde mit 3 Volumina Puffer "W" mit 2.5 mM Desthiobiotin in Puffer "W" eluiert. Nach mehrmaliger Dialyse gegen Puffer "W" und Aufkonzentration wurde über SDS-PAGE und UV-Spektroskopie (Burda et.al. 1999) die Konzentration und Reinheit von N-Strep-T4p12 ermittelt. Aus 10 Liter Kultur wurden so ca. 100 mgN-Strep-T4p12 gereinigt.

| Name | Sequenz des Tag | |
|---|---|---|
| Nstrep-p12 | MASWSHPQFEKGAS | SEQ ID NO: 5 |
| Nstrep-p12-S3C | MACWSHPQFEKGAS | SEQ ID NO: 6 |
| Nstrep-p12-S14C | MASWSHPQFEKGAC | SEQ ID NO: 7 |

5. Alternative Reinigung von p12: Hierzu wurde das Zellpellet (aus 10 l Kultur, BL21 (DE3) transformiert mit dem Plasmid pNS-T4p12p57 oder pT4p12p57) in Puffer 1, (10mM Natriumphosphat pH 9, 500 mM NaCl, 4mM MgCl2) aufgenommen und per Frenchpress (wie unter 3 beschrieben) aufgeschlossen. Anschliessend wurde das Material bei 20000rpm (SS-34) 45 Minuten zentrifugiert und das Pellet in ca. 25ml Puffer 1 resuspendiert (= gewaschen). Dieser Waschschritt wurde zweimal wiederholt und das Pellet mit 25ml Puffer 2: (50mM NaPi pH 5, 100mM NaCl, 25mM EDTA) extrahiert. Hierfür wurde das resuspendierte Pellet zur Extraktion 60 Minuten bei Raumtemperatur gerührt und danach abzentrifugiert (20000rpm (SS-34) für 45 Minuten). Diese Extraktion wurde bei Bedarf 2 mal wiederholt. Im Anschluss wurden die Überstände der Extraktion vereinigt und direkt auf eine Anionentauscher-Säule (ResourceS, Pharmacia) aufgetragen. Als Laufpuffer wurde 15 mM Na Hydrogenphosphat, 15 mM Na Formiat, 30 mM Na Acetat, pH 5.0, 50 mM NaCl verwendet. Die Elution erfolgte über einen linearen Salzgradienten von 50 mM NaCl bis 600 mM NaCl im Laufpuffer. Das gereinigte p12 wurde anschließend zur Lagerung gegen 50 mM Tris pH 8.5, 150 mM NaCl, 5 mM EDTA oder PBS dialysiert, in Aliquots in N₂ gefroren und bei -20°C gelagert.
6. Biotinylierung von p12: Zur Biotinylierung des p12 Proteins wurden entweder EZ-Link^{™}Sulfo-NHS-LC-LC-Biotin oder EZ-Link^{™}TFP-PEO-Biotin von Pierce, USA verwendet und die Biotinylierung nach den vom Hersteller angegebenen Vorschriften durchgeführt. Dabei wurden für die Biotinylierung ca. 30 Moleküle Biotin pro p12-Trimer eingesetzt. Die Kopplung von Biotin wurde anschließend mit dem HABA-Assay (Savage MD, Mattson G, Desai S, Nielander GW, Morgensen S und Conklin EJ, 1992, Avidin-Biotin Chemistry: A Handbook, Pierce, Illinois) überprüft und quantifiziert. Durchschnittlich wurden letztendlich 5-10 Moleküle Biotin pro p12-Trimer gebunden.
7. Zur Biotinylierung des N-terminal eingeführten Cysteins wurde EZ-Link^{™}-PEO-Maleiimid-Biotin und EZ-Link^{™}PEO-Iodoacetyl-Biotin von Pierce, USA nach Vorschrift des Herstellers eingesetzt. Die Reaktion wurde wie unter 5 beschrieben überprüft.
8. Biotinylierung des Bakteriophagen T4: Der Bakteriophage wurde nach Bachrach U und Friedmann A (1971) Practical procedures for the purification of bacterial viruses, Appl Microbiol 22: 706-715, gereinigt. Der gereinigte Phage wurde gegen PBS dialysiert und mit EZ-Link^{™}Sulfo-NHS-LC-LC-Biotin oder EZ-Link^{™}TFP-PEO-Biotin von Pierce, USA nach Angaben des Herstellers in einem Verhältnis von 100-100000 Biotinmolekülen pro Phage markiert.
9. p12-abhängige Ernte von *E.coli* nach dem 1-Schritt-Verfahren und dem 2-Schritt-Verfahren (Fig. 1): Bei der Bindung nach dem 1-Schritt-Verfahren (VC) wurde das N-Strep-p12 Protein 1h mit den magnetischen Streptavidinbeads (10 µg Protein / 50 µl 1% Beads) inkubiert und die Beads wurden anschließend 3x mit PBST gewaschen. Für die Zellbindung wurden je 200 µl einer 1 zu 10 in LB verdünnten *E.coli* ÜN-Kultur *E.coli* Kultur (ca. 1 x 10⁸ Zellen/ml) pro well in einer Mikrotiterplatte mit 10 µl der N-Strep-p12 beschichteten Beads vermischt und für verschiedene Zeiten (Fig. 1) bei RT inkubiert. Anschließend wurden die Beads mit den gebundenen Zellen mittels eines Magnetseparators (Bilatec AG, Mannheim) für 3-5 min an der Wandung der wells konzentriert. Die Beads wurden 3x mit PBST gewaschen und anschließend wurde die β-Galactosidaseaktivität (Apte SC et al., 1995, Water res. 29, 1803-06) der an den Beads verbliebenen Zellen ermittelt. Bei der Bindung nach dem 2-Schritt-Verfahren wurde eine 1 :10 Verdünnung einer *E.coli* ÜN-Kultur *E.coli* Kultur (ca. 1 x 10⁸ Zellen/ml) für 1 h (in weiteren Versuchen für 1 min, 3 min, 10 min, 30 min) mit dem N-Strep-p12 Protein (10 µg Protein / ml Zellsuspension) bei RT inkubiert. Anschließend wurden 200 µl des Protein-Zellgemisches zu 10 µl 1% magnetischer Streptavidinbeads gegeben und für die in der Fig.1 angegebenen Zeiten bei RT inkubiert. Zur Ermittlung der gebundenen Zellen wurde im weiteren wie mit dem 1-Schritt-Verfahren verfahren.
10. T4 abhängige Bindung von *E.coli* nach dem 1-Schritt-Verfahren (VC) und nach dem 2-Schritt-Verfahren (VIK) (Fig. 2). Bei der Bindung nach dem 1-Schritt-Verfahren (VC) wurde der biotinylierte Phage T4 (100 Biotinmoleküle / Phage) für 1 h an 1% Streptavidin-Beads gebunden (10¹⁰ PFU / ml 1% Beads) und die Beads wurden anschließend 3x mit PBST gewaschen. Nach der Zellbindung (25 µl Phagenbeads /ml Zellsuspension) für 2h wurden die Beads gewaschen und die gebundenen *E.coli* über die β-Galactosidaseaktivität nachgewiesen (Angaben in relativen Einheiten). Bei der Bindung nach dem 2-Schritt-Verfahren (VIK) wurden die Zellen mit dem biotinylierten Phagen für 1h inkubiert (2.5 10⁸ PFU / ml Zellsuspension) und die Mischung anschließend für 2h mit den Streptavidin-Beads (25 µl 1% Beads/ ml Zellsuspension) inkubiert. Das weitere Vorgehen war wie beim 1-Schritt-Verfahren. Während der Dauer der Inkubation von Phage und Bakterien wurden dem Medium die Antibiotika Rifampicin (25 µg / ml), Chloramphenicol (25 µg / ml) und Tetracyclin (2 µg / ml) zugesetzt.
11. Ernte von *E.coli* Zellen nach dem 2-Schritt-Verfahren aus verschiedenen Wachstumsmedien (Fig. 3): Die *E.coli* Stämme LE392 und JM83 wurden ÜN in dem jeweiligen Medium angezogen. Zu 200 µl Zellsuspension wurde N-Strep-p12 (10 µg / ml Zellsuspension) gegeben. Nach 5 min Inkubation bei RT wurden 10 µl 1% Streptavidinbeads zugegeben, vermischt durch 3maliges Aufziehen mit der Pipette und weitere 5 min bei RT inkubiert. Anschließend wurden die Bakterien-Beads-Komplexe mittels des o.a. Magnetseparators für 3-5 min abgezogen und die im Überstand verbliebenen Zellen wurden über die Streuung des Überstandes bei 600 nm bestimmt.
12. Plasmidisolierung aus *E.coli* nach Zellernte über das 2-Schritt-Verfahren (Fig. 4). Je 300 µl einer *E.coli* ÜN-Kultur, die das Plasmid pUC19 beinhaltet, wurde nach dem 2-Schritt-Verfahren wie unter 9 beschrieben abgeerntet. Nach Abzug der Zellen mittels des o.a. Magnetseparators wurde aus den Zellen das Plasmid einmal über ein Festphasenextracktionsverfahren (QIAprep, Qiagen, Hilden) und auch über ein Verfahren mit magnetischen Beads (Bilatec, Mannheim) nach den Angaben der Hersteller isoliert.
13. Anreicherung von *E.coli* Zellen aus 10 ml Kulturvolumen über N-Strep-p12 und über den biotinylierten Bakteriophagen T4-bio nach dem 2-Schritt-Verfahren (Fig. 5): Zur Anreicherung mit N-Strep-p12 wurden 10 ml *E.coli* Kulturen mit 10⁹, 10⁸ und 10⁷ Zellen pro ml mit 30 µg, bzw. jeweils 6 µg Protein versetzt und 1h bei 37°C gerollt. Zur Anreicherung mit T4-bio wurden 10 ml Kulturen mit 10⁹, 10⁸ und 10⁷ Zellen pro ml mit jeweils 10¹⁰ T4-bio Phagen und 10 µg Rifampicin / ml und 2 µg Tetracyclin / ml versetzt und 1h bei 37°C gerollt. Anschließend wurde zu den Ansätzen 100 µl 1% magnetische Streptavidinbeads gegeben und für eine weitere Stunde gerollt. Mittels eines Magneten (ABgene, Hamburg) wurden die an die magnetischen Beads gebundenen Zellen abgetrennt, 3x mit PBST gewaschen und über ihre β-Galactosidaseaktivität nachgewiesen.
14. Lebend-Ernte von *E.coli* über das 2-Schritt-Verfahren mit biotinyliertem p12 (Fig. 6): *E.coli* Zellen (200 µl einer Kultur) wurden nach dem 2-Schrittverfahren wie in Beispiel 9 geerntet (10 µg biotinyliertes p12 / ml Zellkultur und 10 µl 1% StreptavidinBeads / ml Zellkultur) und die Beads 2x mit PBST gewaschen. Anschließend wurde die β-Galactosidase-Aktivität bestimmt und das Wachstum der Zellen nach 2h über die Streuung bei 600 nm im Photometer bestimmt.
15. Selektivität der N-Strep-p12-abhängigen Ernte (Fig. 7 und 8): Je 200 µl einer ÜN-Kultur von verschiedenen *E.coli* Stämmen (Fig. 7) sowie von verschiedenen Bakterienstämmen (Fig. 8) wurden nach dem 2-Schritt-Verfahren (wie in Beispiel 9 beschrieben) abgeerntet. Nach Konzentration der Beads im Magnetseparator wurde die abgeerntete Zellmenge über die Streuung des Überstandes bei 600nm bestimmt.
16. Selektive Bindung von *E.coli* an magnetische Streptavidinbeads über T4-bio und Nachweis von *E.coli* über FITC-markiertes p12 (Fig. 9). Das p12 Protein wurde mit FITC (Molecular Probes, Leiden) gemäß den Angaben des Herstellers markiert und gegen PBS dialysiert. Zu einer Mischkultur (ca. 10⁸⁻⁹ Zellen / ml) aus *E.coli* und *Serratia marcescens* wurde FITC-markiertes p12 gegeben (5 µg / ml). Nach 5 min Inkubation bei RT im Dunklen wurden 10⁹ T4-bio Phagen und Rifampicin (10 µg / ml), Chloramphenmicol (25 µg / ml) und Tetracyclin (2 µg /ml) zugegeben. Als Kontrolle wurde nicht biotinylierter T4 Phage eingesetzt. Nach Inkubation für 10 min wurden 1% Streptavidin Beads (10 µl / ml) zugegeben und die Ansätze wurden unter dem Mikroskop betrachtet (Fig. 9).
17. Bestimmung der Nachweisgrenze für die N-Strep-p12 abhängige Isolierung von *E.coli* Zellen nach dem 2-Schritt-Verfahren (Fig. 10): Je 300 µl von Verdünnungen einer *E.coli* ÜN-Kultur (10²-10⁵ Zellen / ml) wurden in Mikrotiterplatten mit N-Strep-p12 (10 µg Protein / ml) für 1h inkubiert. Anschließend wurden magnetische Streptavidinbeads (100 µl 1% Beads / ml) zugegeben, gemischt und die gebundenen Zellen mittels eines Magneten (Bilatek, Mannheim) pelletiert. Die Beads wurden 3x mit PBST gewaschen. Der Nachweis der *E.coli* Zellen erfolgte über Fluoreszenz- und Lumineszenz-Substrate für die β-Galactosidase.
18. Chemische Kopplung von T4p12 an magnetische Beads (Fig. 11): 150 µl 1%iger magnetischer Beads (EM2-100/40, Merck Eurolab, France) wurden 3x mit 10 mM Natriumphosphatpuffer, pH 6 gewaschen und in 40 µl des Puffers aufgenommen. Nach Zugabe von 120 µl EDC-Lösung (1-Ethly-3-(3Dimethylaminopropyl)carbodiimide) (30 mg / ml) und Inkubation für 5 min bei RT wurden 160 µl T4p12 Lösung (0.7 mg Protein / ml 10 mM Natriumphosphatpuffer, pH 6) zupipettiert, gemischt und der Ansatz 2 h bei RT inkubiert. Durch Zumischung von 1 Volumen 0.2 M Tris-HCl, pH 7, 0.05% Tween 20, wurde die Reaktion ÜN bei 4°C abgestoppt. Anschließend wurden die Beads 4x mit PBST gewaschen und mit PBST auf 1% eingestellt. Die Kopplung von p12 an die Beads wurde mittels eines p12-spezifischen Antiserums getestet. Die Bindeaktivität der p12-Beads wurde über die Bindung von *E.coli* Zellen nach dem 1-Schritt-Verfahren ermittelt. Hierfür wurden 3 µl 1% p12-Beads mit 200 µl einer hundertfach verdünnten *E.coli* ÜN-Kultur (ca. 1 x 10⁷ Zellen/ml) 5 min inkubiert, 3x mit PBST gewaschen und die gebundenen Zellen wurden über ihre β-Galactosidaseaktivität nachgewiesen (Fig. 11).
19. Adsorption von p12 an verschiedene magnetische Polyvinylalkohol (PVA) Beads (Fig. 12): 200 µl 2%ige PVA Beads (Chemagen AG, Baesweiler) wurden mit unterschiedlichen Mengen T4p12 (0-5 µg Protein / mg Beads) in 100 mM TrisHCl, pH 8, 1 mM EDTA, 200 mM NaCl ÜN bei 37°C inkubiert. Anschließend wurden die Beads 2x mit PBST gewaschen und in PBS auf 2% aufgenommen. Die funktionale Bindung von T4p12 an die Beads wurde über die Bindung von *E.coli* Zellen ermittelt. Hierzu wurden 200 µl einer *E.coli* ÜN-Kultur mit 10 µl p12-Beads vermischt und 5 min bei RT inkubiert. Nach Abzug der gebundenen Zellen wurde die Streuung des Überstandes bei 600 nm vermessen und zur Streuung der *E.coli* Kultur vor Zugabe der Beads in Relation gesetzt.

## Patentansprüche

1. Verfahren zur selektiven Aufreinigung von Bakterienzellen oder bakteriellen Zellbestandteilen, umfassend die folgenden Schritte:
a) In Kontakt bringen von einer Bakterienzellen oder bakterielle Zellbestandteile enthaltenden Probe mit Bakteriophagenhüllproteinen und/oder Bakteriophagenschwanzproteinen,
b) anschließende Inkubation der Probe, enthaltend die Bakterienzellen oder bakteriellen Zellbestandteile und die Bakteriophagenhüllproteine und/oder Bakteriophagenschwanzproteinen, mit einem festen Träger, wobei der feste Träger eine oder mehrere verschiedene auf seiner Oberfläche die Bakterien und/oder die Bakteriophagenhüllproteine und/oder Bakteriophagenschwanzproteinen bindende Kopplungsgruppe(n) aufweist,
c) Trennen des festen Trägers mit den daran über die Bakteriophagenhüllproteine und/oder Bakteriophagenschwanzproteinen gebundenen Bakterienzellen oder bakteriellen Zellbestandteilen von der Probe.

2. Verfahren nach Anspruch 1, wobei die Kopplungsgruppe ein Lektin, Rezeptor oder Anticalin ist.

3. Verfahren nach Anspruch 1, wobei die Kopplungsgruppe ein Streptavidin oder Avidin ist und die Bakteriophagenhüllproteine und/oder Bakteriophagenschwanzproteinen mit Biotin oder einem Strep-Tag gekoppelt sind.

4. Verfahren nach einem der Ansprüche 1 bis 3, wobei der feste Träger ein Magnetpartikel, Agarosepartikel, Glaspartikel, Luminexpartikel, Reaktionsgefäß oder eine Mikrotiterplatte ist.

5. Verfahren nach einem der Ansprüche 1 bis 4, wobei zwei oder mehr verschiedene Bakteriophagenhüllproteine und/oder Bakteriophagenschwanzproteinen zugegeben werden.

6. Verfahren nach einem der vorhergehenden Ansprüche, wobei der selektiv aufzureinigende bakterielle Zellbestandteil Endotoxin ist.

7. Magnetpartikel beschichtet mit isolierten Bakteriophagenhüllproteinen und/oder Bakteriophagenschwanzproteinen.

8. Magnetpartikel nach Anspruch 7, wobei die die Bakteriophagenhüllproteine und/oder Bakteriophagenschwanzproteinen ausgewählt sind aus der Gruppe
0c1r, 10tur, L2, L51, M1, MVG51, MV-L1, O1, SpV1, V1, V1, V2, V4, V5, 108/016, 119, 29, 37, 43, 51, 59.1, A1-Dat, Aeh2, Bir, M1, MSP8, ø115-A, ø150A, ø31C, P-a-1, PhiC, R1, R2, SK1, SV2, VP5, Ap3, Ap4, Mm1, Mm3, Mm4, Mm5, phiUW 51, 43, 44RR2.8t, 65, Aeh1, PM1, PIIBNV6, PS8, psi, PT11, 8764, A5/A6, A6, PM2, W11, W2, W4, W7, 1A, alpha, AP50, BLE, F, G, GA-1, II, IPy-1, mor1, MP13, MP15, ø105, ø29 (phi 29), øNS11, PBP1, PBS1, SP10, SP15, SP3, SP8, SPP1, SPβ, SPy-2, SST, type,168, W23, SP50, W23, SP01, MAC-1, MAC-2, MAC-4, MAC-5, MAC-7, Tb, øCb12r,øCb2, øCb23r, øCb4, øCb5, øCb8r, øCb9, øCP18, øCP2, øCr14, øCr28, O11, O13, O2, O3, O5, O6, O8, 1, .phi.CPG1, Ceβ, F1, HM2, HM3, HM7, 7/26, A, A19, AN25S-1, Arp, AS-1, BL3, CONX, MT, N1, øA8010, S-6(L), β,A-4(L), AC-1, LPP-1, S-2L, S-4L, SM-1, P1, T1, Tula, Tulb, Tull,1ø3, 1ø7, 1ø9, 2D/13, Ae2, alpha10, alpha3, BE/1, BF23, dA, delta1, delta6, dø3, dø4, dø5, Ec9, eta8, f1, fd, G13, G14, G4, G6, HK022, HK97, HR, lambda, M13, M13mp18, M20, MM, MS2, Mu, O1, ø80, øA, øR, øX174, PA-2, P1, P1D, P2, P22, Qβ, R17, S13, St-1, T1, T2, T3, T4, T5, T6, T7, WA/1, WF/1, WW/1, zeta3, ZG/2, ZJ/2, C21, omega 8, U3, chi, FC3-9, µ2, 01, 11F, 121, 1412, 3, 3T+, 50, 5845, 66F, 7480b, 8893, 9, 9266, a1, alpha15, b4, B6, B7, Beccles, BZ13, C-1, C16, C2, C-2, DdVI, Esc-7-11, f2, fcan, FI, Folac, fr, GA, H, H-19J, 12-2, lalpha, ID2, If1, If2, Ike, JP34, JP501, K19, KU1, M, M11, M12, MS2, NL95, ø92, øl, Øii, Omega8, pilHalpha, PR64FS, PRD1, PST, PTB, R, R17, R23, R34, sd, SF, SMB, SMP2, SP, β, ST, tau, tf-1, TH1, TW18, TW28, Vill, VK, W31, X, Y, ZG/1, ZIK/1, ZJ/1, ZU3, ZS/3, AP3, C3:, 1b6, 223, fri, hv, hw222a, øFSW, PL-1, y5, 1, 643, c2, kh, m13, P008, P127, 1358, 1483, 936, 949, BK5-T, c2, KSY1, P001, P008, P107, P335, P034, P087, pro2, 4211, psi M2 (ΨM2), N1, N5, Br1, C3, L3, I3, lacticola, Leo, ø17, R1-Myb, N13, N18, N24, N26, N36, N4, N5, X1, X10, X24, X3, X5, X6, D3, D4, 22, 32, AU, C-2, P1, P2, P3, P4, Phi CT, phi CTX, PB-1, 12S, 7s, D3, F116, gh-1, gh-1, Kf1, M6, ø1, øKZ, øW-14, Pf1, W3, 2, 16-2-12, 2, 317, 5, 7-7-7, CM1, CT4, m, NM1, NT2, ø2037/1, ø2042, øgal-1-R, WT1, Mp1, MP2, W1, epsilon15, Felix 01, 16-19, 7-11, H-19J, Jersey, N4, SasL1, Vil, ZG/3A, San21, A3, A4, P22, 4, C1/TS2, Sp1, 107, 187, 2848A, 3A, 44AHJD, 6, 77, B11-M15, Twort, 182, 2BV, A25, A25-24, A25-omega8, A25-PE1, A25-VD13, CP-1, Cvir, H39, P23, P26, phi A.streptomycini III, phi8238, phiC31, S1, S2, S3, S4, S6, S7, SH10, Tb1, Tb2, Ts1, 06N-22P, 06N-58P, 06N-58P, 4996, alpha3alpha, I, II, III, IV, kappa, nt-1, OXN-100P, OXN-52P, v6, Vf12, Vf33, VP1, VP11, VP3, VP5, X29, Cf, Cf1t, RR66, 8/C239, phiYe03-12, YerA41.

9. Magnetpartikel nach Anspruch 7 oder 8 mit einem Durchmesser von etwa 0,5 bis etwa 4 µm oder etwa 0,8 bis etwa 1,8 µm.

10. Bakteriophagenhüllprotein oder Bakteriophagenschwanzprotein umfassend einen Tag, wobei der Tag eine Aminosäuresequenz gemäß SEQ ID NO: 5, 6 oder 7 aufweist.

11. Bakteriophagenschwanzprotein nach Anspruch 10, wobei das Bakteriophagenschwanzprotein das p12-Protein des Phagen T4 ist.

12. Bakteriophagenhüllprotein oder Bakteriophagenschwanzprotein nach einem der Ansprüche 10 bis 11, wobei das Bakteriophagenhüllprotein oder das Bakteriophagenschwanzprotein mittels DNA-Rekombinationstechnik hergestellt ist.

13. Nukleinsäure codierend ein Bakteriophagenhüllprotein oder ein Bakteriophagenschwanzprotein nach einem der Ansprüche 10 bis 12.

14. Aminosäure mit einer Sequenz gemäß SEQ ID NO: 6, 7 oder 8.

15. Verwendung der Magnetpartikel nach einem der Ansprüche 7 bis 9 oder der Bakteriophagenhüllproteine und/oder der Bakteriophagenschwanzproteine nach einem der Ansprüche 10 bis 12 zur Isolierung von Nukleinsäuren, insbesondere zur Plasmidpräparation, von bakteriellen Zellbestandteilen, insbesondere von Lipopolysacchariden, Endotoxinen oder Exopolysacchariden.

16. Kit zur Aufreinigung von Bakterienzellen und/oder bakteriellen Zellbestandteilen, umfassend die Magnetpartikel nach einem der Ansprüche 7 bis 9 und/oder der Bakteriophagenhüllproteine und/oder Bakteriophagenschwanzproteine nach einem der Ansprüche 10 bis 12.

## Claims

1. Method for the selective purification of bacterial cells or cell components, comprising the following steps:
a) contacting a sample containing bacterial cells or bacterial cell components with bacteriophage coat proteins and/or bacteriophage tail proteins,
b) subsequent incubation of the sample, containing the bacterial cells or bacterial cell components and the bacteriophage coat proteins and/or bacteriophage tail proteins, with a solid support, wherein the solid support exhibits one or more different coupling group(s) on its surface, binding the bacteria and/or bacteriophage coat proteins and/or bacteriophage tail proteins,
c) separating the solid support with the bacterial cells or bacterial cell components bound thereto via the bacteriophage coat proteins and/or bacteriophage tail proteins from the sample.

2. Method according to claim 1, wherein the coupling group is a lectin, receptor or anticalin.

3. Method according to claim 1, wherein the coupling group is a streptavidin or avidin and the bacteriophage coat proteins and/or bacteriophage tail proteins are coupled with biotin or a strep-tag.

4. Method according to any one of claims 1 to 3, wherein the solid support is a magnetic particle, agarose particle, glass particle, luminex particle, reaction tube, or a microtiter plate.

5. Method according to any one of claims 1 to 4, wherein two or more different bacteriophage coat proteins and/or bacteriophage tail proteins are added.

6. Method according to any one of the preceding claims, wherein the bacterial cell component to be selectively purified is Endotoxin.

7. Magnetic particle coated with isolated bacteriophage coat proteins and/or bacteriophage tail proteins.

8. Magnetic particle according to claim 7, wherein the bacteriophage coat proteins and/or bacteriophage tail proteins are selected from the group
0c1r, 10tur, L2, L51, M1, MVG51, MV-L1, O1, SpV1, V1, V1, V2, V4, V5, 108/016, 119, 29, 37, 43, 51, 59.1, A1-Dat, Aeh2, Bir, M1, MSP8, ø115-A, ø150A, ø31C, P-a-1, PhiC, R1, R2, SK1, SV2, VP5, Ap3, Ap4, Mm1, Mm3, Mm4, Mm5, phiUW 51, 43, 44RR2.8t, 65, Aeh1, PM1, PIIBNV6, PS8, psi, PT11, 8764, A5/A6, A6, PM2, W11, W2, W4, W7, 1A, alpha, AP50, BLE, F, G, GA-1, II, IPy-1, mor1, MP13, MP15, ø105, ø29 (phi 29), øNS11, PBP1, PBS1, SP10, SP15, SP3, SP8, SPP1, SPβ, SPy-2, SST, type,168, W23, SP50, W23, SP01, MAC-1, MAC-2, MAC-4, MAC-5, MAC-7, Tb, øCb12r,øCb2, øCb23r, øCb4, øCb5, øCb8r, øCb9, øCP18, øCP2, øCr14, øCr28, O11, O13, O2, O3, O5, O6, O8, 1, .phi.CPG1, Ceβ, F1, HM2, HM3, HM7, 7/26, A, A19, AN25S-1, Arp, AS-1, BL3, CONX, MT, N1, øA8010, S-6(L), β,A-4(L), AC-1, LPP-1, S-2L, S-4L, SM-1, P1, T1, TuIa, TuIb, TuII,1ø3 , 1ø7, 1ø9, 2D/13, Ae2, alpha10, alpha3, BE/1, BF23, dA, deltal, delta6, dø3, dø4, dø5, Ec9, eta8, f1, fd, G13, G14, G4, G6, HK022, HK97, HR, lambda, M13, M13mp18, M20, MM, MS2, Mu, O1, ø80, øA, øR, øX174, PA-2, P1, P1D, P2, P22, Qβ, R17, S13, St-1, T1, T2, T3, T4, T5, T6, T7, WA/1, WF/1, WW/1, zeta3, ZG/2, ZJ/2, C21, omega 8, U3, chi, FC3-9, µ2, 01, 11F, 121, 1412, 3, 3T+, 50, 5845, 66F, 7480b, 8893, 9, 9266, al, alpha15, b4, B6, B7, Beccles, BZ13, C-1, C16, C2, C-2, DdVI, Esc-7-11, f2, fcan, FI, Folac, fr, GA, H, H-19J, I2-2, Ialpha, ID2, If1, If2, Ike, JP34, JP501, K19, KU1, M, M11, M12, MS2, NL95, ø92, øI, Øii, Omega8, pilHalpha, PR64FS, PRD1, PST, PTB, R, R17, R23, R34, sd, SF, SMB, SMP2, SP, β, ST, tau, tf-1, TH1, TW18, TW28, ViII, VK, W31, X, Y, ZG/1, ZIK/1, ZJ/1, ZL/3, ZS/3, AP3, C3:, 1b6, 223, fri, hv, hw222a, øFSW, PL-1, y5, 1, 643, c2, kh, m13, P008, P127, 1358, 1483, 936, 949, BK5-T, c2, KSY1, P001, P008, P107, P335, PO34, PO87, pro2, 4211, psi M2 (ΨM2), N1, N5, Br1, C3, L3, I3, lacticola, Leo, ø17, R1-Myb, N13, N18, N24, N26, N36, N4, N5, X1, X10, X24, X3, X5, X6, D3, D4, 22, 32, AU, C-2, P1, P2, P3, P4, Phi CT, phi CTX, PB-1, 12S, 7s, D3, F116, gh-1, gh-1, Kf1, M6, ø1, øKZ, øW-14, Pfl, W3, 2, 16-2-12, 2, 317, 5, 7-7-7, CM1, CT4, m, NM1, NT2, ø2037/1, ø2042, øgal-1-R, WT1, Mp1, MP2, W1, epsilon15, Felix 01, 16-19, 7-11, H-19J, Jersey, N4, SasL1, ViI, ZG/3A, San21, A3, A4, P22, 4, C1/TS2, Sp1, 107, 187, 2848A, 3A, 44AHJD, 6, 77, B11-M15, Twort, 182, 2BV, A25, A25-24, A25-omega8, A25-PE1, A25-VD13, CP-1, Cvir, H39, P23, P26, phi A.streptomycini III, phi8238, phiC31, S1, S2, S3, S4, S6, S7, SH10, Tb1, Tb2, Ts1, 06N-22P, 06N-58P, 06N-58P, 4996, alpha3alpha, I, II, III, IV, kappa, nt-1, OXN-100P, OXN-52P, v6, Vf12, Vf33, VP1, VP11, VP3, VP5, X29, Cf, Cf1t, RR66, 8/C239, phiYeO3-12, YerA41.

9. Magnetic particle according to claims 7 or 8, having a diameter of about 0.5 to about 4 µm or about 0.8 to about 1.8 µm.

10. Bacteriophage coat protein or bacteriophage tail protein comprising a tag, whereby the tag exhibits an amino acid sequence according to SEQ ID NO: 5, 6 or 7.

11. Bacteriophage protein according to claim 10, whereby the bacteriophage tail protein is the p12 protein of the phage T4.

12. Bacteriophage protein according to claim 10 or 11, whereby the bacteriophage coat protein or the bacteriophage tail protein is produced via DNA recombination technology.

13. Nucleic acid encoding a bacteriophage coat protein or a bacteriophage tail protein according to any one of the claims 10 to 12.

14. Amino acid having a sequence according to SEQ ID NO: 6, 7 or 8.

15. Use of the magnetic particles according to any one of the claims 7 to 9 or the bacteriophage coat proteins or the bacteriophage tail proteins according to any one of the claims 10 to 12 for the isolation of nucleic acids, in particular for the plasmid preparation, of bacterial cell components, in particular of lipopolysaccharides, endotoxines or exopolysaccharides.

16. Kit for the purification of bacterial cells and/or bacterial cell components, comprising the magnetic particles according to any one of claims 7 to 9 and/or the bacteriophage coat proteins or the bacteriophage tail proteins according to any one of claims 10 to 12.

## Revendications

1. Procédé pour la purification sélective de cellules bactériennes ou de composants cellulaires bactériens, comprenant les étapes suivantes :
a) mise en contact d'un échantillon comprenant des cellules bactériennes ou des composants cellulaires bactériens avec des protéines de capsides de bactériophages et/ou des protéines de queues de bactériophages,
b) suivie d'une incubation de l'échantillon, comprenant les cellules bactériennes ou les composants cellulaires bactériens et des protéines de capsides de bactériophages et/ou des protéines de queues de bactériophages, avec un support solide, le support solide présentant un ou plusieurs groupes de couplage à sa surface se liant aux bactéries et/ou aux protéines de capsides de bactériophages et/ou aux protéines de queues de bactériophages,
c) séparation de l'échantillon du support solide portant les cellules bactériennes ou les composants cellulaires bactériens liés à celui-ci par les protéines de capsides de bactériophages et/ou les protéines de queues de bactériophages.

2. Procédé selon la revendication 1, dans lequel le groupe de couplage est une lectine, un récepteur ou une anticaline.

3. Procédé selon la revendication 1, dans lequel le groupe de couplage est une streptavidine ou une avidine et les protéines de capsides de bactériophages et/ou les protéines de queues de bactériophages sont couplées avec une biotine ou un strep-tag.

4. Procédé selon l'une quelconque des revendications 1 à 3, dans lequel le support solide est constitué de particules magnétiques, de particules d'agarose, de particules de verre, de particule de Luminex, d'un récipient à réaction ou d'une plaque de microtitrage.

5. Procédé selon l'une quelconque des revendications 1 à 4, dans lequel deux protéines de capsides de bactériophages et/ou protéines de queues de bactériophages ou plus sont ajoutées.

6. Procédé selon l'une quelconque des revendications précédentes, dans lequel le composant cellulaire bactérien à purifier sélectivement est une endotoxine.

7. Particules magnétiques recouvertes de protéines de capsides de bactériophages et/ou de protéines de queues de bactériophages isolées.

8. Particules magnétiques selon la revendication 7, les protéines de capsides de bactériophages et/ou les protéines de queues de bactériophages étant choisies dans le groupe
0c1r, 10tur, L2, L51, M1, MVG51, MV-L1, O1, SpV1, V1, V1, V2, V4, V5, 108/016,119, 29, 37, 43, 51, 59.1, A1-Dat, Aeh2, Bir, M1, MSP8, Ø115-A, Ø150A, Ø31C, P-a-1, PhiC, R1, R2, SK1, SV2, VP5, Ap3, Ap4, Mm1, Mm3, Mm4, Mm5, phiUW 51, 43, 44RR2.8t, 65, Aeh1, PM1, PIIBNV6, PS8, psi, PT11, 8764, A5/A6, A6, PM2, W11, W2, W4, W7, 1A, alpha, AP50, BLE, F, G, GA-1, II, IPy-1, mor1, MP13, MP15, Ø105, Ø29 (phi 29), ØNS11, PBSI, PBS1, SP10, SP15, SP3, SP8, SPP1, SPβ, SPy-2, SST, type, 168, W23, SP5O, W23, SP01, MAC-1, MAC-2, MAC-4, MAC-5, MAC-7, Tb, ØCb12r, ØCb2, ØCb23r, ØCb4, ØCb5, ØCb8r, ØCb9, ØCP18, ØCP2, ØCr14, ØCr28, O11, O13, O2, O3, O5, O6, O8, 1, phi. CPG1, Ceβ, F1, HM2, HM3, HM7, 7/26, A, A19, AN25S-1, Arp, AS-1, BL3, CONX, MT, N1, ØA8010, S-6(L), β,A-4(L), AC-1, LPP-1, S-2L, S-4L, SM-1, P1, T1, Tula, Tulb, Tull, 1Ø3, 1Ø7, 1Ø9, 2D/13, Ae2, alpha10, alpha3, BE/1, BF23, dA, delta1, delta6, dØ3, dØ4, dØ5, Ec9, eta8, fi, fd, G13, G14, G4, G6, HK022, HK97, HR, lambda, M13, M13mp18, M20, MM, MS2, Mu, O1, Ø80, ØA, ØR, ØX174, PA-2, P1, P1D, P2, P22, Oβ, R17, S13, St-1, T1, T2, T3, T4, T5, T6, T7, WA/1, WF/1, WW/1, zeta3, ZG/2, ZJ/2, C21, omega 8, U3, chi, FC3-9, µ2, 01, 11F, 121, 1412, 3, 3T+, 50, 5845, 66F, 7480b, 8893, 9, 9266, al, alpha15, b4, B6, B7, Beccles, BZ13, C-1, C16, C2, C-2, DdVI, Esc-7-11, f2, fcan, FI, Folac, fr, GA, H, H-19J, I2-2, lalpha, ID2, lfl, fl2, lke, JP34, JP501, K19, KU1, M, M11, M12, MS2, NL95, Ø92, Øi, Ø11, Omega8, pilHalpha, PR64FS, PRD1, PST, PTB, R, R17, R23, R34, sd, SF, SMB, SMP2, SP, β, ST, tau, tf-1, TH1, TW18, TW28, Vit1, VK, W31, X, Y, ZG/1, ZIK/1, ZJ/1, ZL/3, ZS/3, AP3, C3:, 1b6, 223, fri, hv, hw222a, ØFSW, PL-1, y5, 1, 643, c2, kh, m/3, P008, P127, 1358, 1483, 936, 949, BK5-T, c2, KSY1, P001, P008, P107, P335, P034, P087, pro2, 4211, psi M2 (ψM2), NI, N5, Br1, C3, L3, I3, lacticola, Leo, Ø17, Ri-Myb, N13, N18, N24, N26, N36, N4, N5, X1, X10, X24, X3, X5, X6, D3, D4, 22, 32, AU, C-2, P1, P2, P3, P4, Phi CT, phi CTX, PB-1, 12S, 7s, D3, F116, gh-1, gh-1, Kf1, M6, Ø1, ØKZ, ØW-14, Pfl, W3, 2, 16-2-12, 2, 317, 5, 7-7-7, CM1, CT4, m, NM1, NT2, Ø2037/1, Ø2042, Øgal-1-R, WT1, Mp1, MP2, W1, epsilon 15, Felix 01, 16-19, 7-11, H-19J, Jersey, N4, SasL1, Vil, ZG/3A, San21, A3, A4, P22, 4, C1/TS2, Sp1, 107, 187, 2848A, 3A, 44AHJD, 6, 77, B11-M15, Twort, 182, 2BV, A25, A25-24, A25-omega8, A25-PE1, A25-VD13, CP-1, Cvir, H39, P23, P26, phi A.streptomycini III, phi8238, phiC31, S1, S2, S3, S4, S6, S7, SH10, Tb1, Tb2, Ts1, 06N-22P, 06N-58P, 06N-58P, 4996, alpha3alpha, I, II, III, IV, kappa, nt-1, OXN-100P, OXN-52P, v6, Vf12, Vf33, VP1, VP11, VP3, VP5, X29, Cf, Cf1t, RR66, 8/C239, phiYeO3-12, YerA41.

9. Particules magnétiques selon la revendication 7 ou 8 présentant un diamètre d'environ 0,5 à environ 4 µm ou d'environ 0,8 à environ 1,8 µm.

10. Protéine de capside de bactériophage ou protéine de queue de bactériophage comprenant une étiquette (tag), l'étiquette présentant une séquence d'acides aminés selon SEQ ID NO : 5, 6 ou 7.

11. Protéine de queue de bactériophage selon la revendication 10, dans laquelle la protéine de queue de bactériophage est la protéine p12 du phage T4.

12. Protéine de capside de bactériophage ou protéine de queue de bactériophage selon l'une quelconque des revendications 10 à 11, dans laquelle la protéine de capside de bactériophage ou la protéine de queue de bactériophage est élaborée par génie génétique.

13. Acide nucléique codant pour une protéine de capside de bactériophage ou une protéine de queue de bactériophage selon l'une quelconque des revendications 10 à 12.

14. Acides aminés présentant une séquence selon SEQ ID NO : 6, 7 ou 8.

15. Utilisation de particules magnétiques selon l'une quelconque des revendications 7 à 9 ou de protéines de capsides de bactériophages et/ou de protéines de queues de bactériophages selon l'une quelconque des revendications 10 à 12 pour l'isolement d'acides nucléiques, en particulier pour la préparation de plasmides, de composants cellulaires bactériens, en particulier de lipopolysaccharides, d'endotoxines ou d'exopolysaccharides.

16. Kit pour la purification de cellules bactériennes et/ou de composants cellulaires bactériens, comprenant les particules magnétiques selon l'une quelconque des revendications 7 à 9 et/ou les protéines de capsides de bactériophages et/ou les protéines de queues de bactériophages selon l'une quelconque des revendications 10 à 12.
